# EUROPÄISCHE PATENTANMELDUNG

(11) **EP 3 603 577 A1**
(43) Veröffentlichungstag der Anmeldung: **05.02.2020**
(21) Anmeldenummer: 18186320.0
(22) Anmeldetag: 30.07.2018
(51) Int. Cl.: A61F 2/30, A61L 27/00, B05D 5/08, B05D 3/10

(54) **INERTISIERUNG VON MATERIALOBERFLÄCHEN DURCH FUNKTIONALISIERTE PERFLUORIERTE MOLEKÜLE**

(71) Anmelder: Technische Universität Berlin, 10623 Berlin (DE)
(72) Erfinder: Schäfer, Konstanze, 10405 Berlin (DE); John-Müller, Astrid, 13507 Berlin (DE); Krämer, Steffi, 13349 Berlin (DE)
(74) Vertreter: Hertin und Partner Rechts- und Patentanwälte PartG mbB

(57) **Zusammenfassung**

Die Erfindung betrifft bevorzugt ein Verfahren zur Inertisierung von Materialoberflächen, bevorzugt von Keramik-, Metall oder Kunststoffoberflächen mittels funktionalisierter perfluorierter Verbindungen. Die dadurch hergestellten oder herstellbaren inerten Oberflächen besitzen eine extrem niedrige Oberflächenenergie, sind resistent gegen Ablagerungen von Substanzen oder von Zellen und besitzen einen sehr niedrigen Reibungskoeffizienten. Weiterhin betrifft die Erfindung praktische Anwendungsmöglichkeiten der inerten Oberflächen.

## Beschreibung

Die Erfindung betrifft bevorzugt ein Verfahren zur Inertisierung von Materialoberflächen, bevorzugt von Keramik-, Metall oder Kunststoffoberflächen mittels funktionalisierter perfluorierter Verbindungen. Die dadurch hergestellten oder herstellbaren inerten Oberflächen besitzen eine extrem niedrige Oberflächenenergie, sind resistent gegen Ablagerungen von Substanzen oder von Zellen und besitzen einen sehr niedrigen Reibungskoeffizienten. Weiterhin betrifft die Erfindung praktische Anwendungsmöglichkeiten der inerten Oberflächen.

### Hintergrund der Erfindung und Stand der Technik

Keramiken und Hochleistungskeramiken sowie Metalle besitzen in der Regel relativ hohe Oberflächenenergien. Auch viele Kunststoffe besitzen aufgrund des Vorhandenseins von Heteroatomen oder funktionellen Gruppen relativ hohe Oberflächenenergien. Andere Kunststoffe wiederum besitzen aufgrund ihres hydrophoben Aufbaus relativ geringe Oberflächenenergien. Allen diesen Kunststofftypen ist jedoch gemein, dass sich in der Regel Substanzen oder Zellen auf der Oberfläche ablagern können.

Viele Anwendungen in der Industrie, in der Medizin bzw. Medizintechnik, in der Elektrotechnik bzw. Elektronik oder in anderen Bereichen erfordern inerte Oberflächen, an denen eine Materialanlagerung vermieden wird oder welche besonders geringe Reibungskoeffizienten besitzen. In diesen Bereichen finden Kunststoffe seit der Weiterentwicklung des 3D-Drucks immer mehr an Bedeutung, wobei die nicht inerten Eigenschaften zu unerwünschten Effekten führen.

Beispielsweise führt im medizinischen bzw. medizintechnischen Bereich eine relativ hohe Oberflächenenergie für Keramiken, Metalle oder Kunststoffe zur Anlagerung von Zellen. In einigen dieser Anwendungen beispielsweise im Bereich medizinischer Implantate soll die Anlagerung von Zellen, insbesondere eine unspezifische Zellanlagerung, unterbunden werden.

Es besteht somit ein großes Bedürfnis, die entsprechenden Oberflächen derart zu modifizieren, dass diese gegenüber der Umwelt weitestgehend inert sind. Insbesondere ist es wünschenswert, dass die Oberflächen der Medizinprodukte keine oder nur eine sehr geringe Wechselwirkung mit lipophilen oder hydrophilen Substanzen eingehen, so dass an der Oberfläche keine Zellanlagerung oder die Anlagerung von Substanzen stattfindet. Beispielsweise kann es bei einer Herzmaschine an nicht hinreichend inerten Oberflächen zur Ablagerung von Gerinnungsfaktoren und Gerinnungsproteinen kommen, die zur Plaque-Bildung führen. Die Ablösung solcher Plaques kann Herzinfarkte oder Schlaganfälle bedingen und stellt somit ein hohes medizinisches Risiko dar. Auch soll oft eine Reibung zwischen zwei Oberflächen vermindert werden.

Insbesondere für biologische Anwendungen offenbart die WO 2012/100100A2 ein Verfahren, in dem auf eine Oberfläche eine Beschichtung aufgebracht wird, die aus einem polyfluorierten oder perfluorierten Polymer besteht (z.B. Teflon). Diese Beschichtung haftet durch Adhäsion. In einem zweiten Schritt werden flüssige Perfluorocarbone (PFCs) ohne funktionelle Gruppen (also PFCs die in der Regel nur aus C und aus F bestehen) auf die erste Schicht aufgetragen bzw. hybridisiert. Diese zweite Schicht hält ebenfalls über Adhäsion, da sich die PFCs in wässriger Umgebung sehr lipophil verhalten und sich an die lipophilen Strukturen der ersten Schicht anlagern. Dadurch wird eine inerte Oberfläche hergestellt.

Während sich das in der WO 2012/100100 A2 beschriebene Verfahren durch eine einfache Herstellung auszeichnet, liegen die PFCs nur durch Adhäsion auf der Oberfläche auf. Dies führt mit der Zeit zu deren Ablösung. Es ist bekannt, dass sich PFCs nur mit bevorzugt gleichem Typ mischen, verschiedene PFCs untereinander entmischen sich nach einem bestimmten Zeitraum. Je ähnlicher PFCs untereinander sind, desto länger dauert es bis zur Entmischung. Je verschiedener die PFCs in einem Gemisch sind, desto schneller entmischen sie. Die Wechselwirkungen zwischen den Molekülen der flüssigen aufgetragen PFC-Schicht untereinander sind daher größer als deren Wechselwirkungen zu der ersten (z.B.Teflon-)Schicht, die zwischen Untergrund und den flüssigen PFCs vermitteln soll. Es ist somit zu erwarten, dass sich mit der Zeit verschiedene Phasen ausbilden, wobei durch eine Agglomeratsbildung die homogene flüssige PFC-Schicht zerstört wird und die inerten Eigenschaften beeinträchtigt. Den in der WO 2012/1001 00A2 hergestellten inerten Schichten mangelt es mithin an einer langen Haltbarkeit.

Inertisierungen von Kunststoffoberflächen weisen derzeit im Stand der Technik eine Reihe von Mängeln auf. Für Kunststoffoberflächen ist beispielsweise eine Begasung mit Fluorwasserstoff (HF) bekannt. Hier kommt es zwar zu einer Inertisierung, die aber nicht sehr effektiv ist, weil nur einzelne Wasserstoffatome (H) gegen einzelne Fluoratome (F) ausgetauscht werden.

Ebenfalls bekannt ist die Beschichtung von Kunststoffoberflächen mit polyfluorierten oder perfluorierten Polymeren (Kunststoffen) wie beispielsweise Teflon. Auch bei den poly- und perfluorierten Polymeren sind nur einzelne Wasserstoffatome durch einzelne Fluoratome ausgetauscht. Ähnlich wie bei der Behandlung mit Fluorwasserstoff sind die dadurch erzielten perfluorierten Strukturen klein und die die erzielten inerten Eigenschaften vergleichsweise zur Anbindung von perfluorierten Ketten gering.

Eine Einbindung von perfluorierten Verbindungen bereits während der Kunststoffherstellung (z.B. durch Pfropf- oder Copolymere, Blends) ist zwar denkbar, kann jedoch zu unerwünschten mechanische Eigenschaften, u.a. in Bezug auf die Abriebsfestigkeit, führen. Beispielsweise mussten Implantate mit einer Teflonbeschichtung nach wenigen Jahren entfernt werden, weil abgeriebene Partikel im Patienten zu Entzündungen führten

Eine Anbindung perfluorierter Verbindungen an Kunststoffoberflächen ist für das Analyseverfahren XPS (Photoelektronenspektroskopie) bekannt. ("Chemische Derivatisierung zur Quantifizierung von funktionellen Gruppen", J. Friedrich, R. Mix, A. Meyer-Plath, S. Hanelt). Hier werden nicht detektierbare funktionelle Gruppen von Kunststoffen in ein Fluorderivat überführt, um mit Hilfe einer Fluor-Konzentrationsbestimmung die ansonsten nicht eindeutig quantifizierbaren funktionellen Gruppen bestimmen zu können. Eine Inertisierung erfolgt hierdurch nicht.

Auch im Bereich der Werkzeugtechnik für Keramik- und Metalloberflächen insbesondere bei hohen mechanischen Belastungen beispielsweise für Tablettierwerkzeuge sind Antihaftbeschichtungen mit verschleißfesten Oberflächen wünschenswert.

Im Stand der Technik ist es daher bekannt Oberflächenmodifikationen anzustreben, welche besonders inert sind, also eine geringe Haftung ggü. Fremdmaterialien und gleichzeitig hohe Abriebsfestigkeit aufweisen.

In einer Aufstellung des Fraunhofer Instituts für Schicht- und Oberflächentechnik IST in Braunschweig (https://www.ist.fraunhofer.de/content/dam/ist/de/documents/produktblatt/Produktblatt_V erschlei%C3%9Ffeste_Antihaftschichten.pdf) wurden marktübliche Beschichtungen zur Verringerung der Oberflächenenergie verglichen:

| Beschichtungen | Oberflächenenergie [mN/m] | Härte[GPa] | Reibkoeffizient (gegen Stahl, trocken) |
|---|---|---|---|
| DLC (a-C:H) | 35 - 40 | 20 - 30 | 0,10 - 0,20 |
| Me-DLC (a-C:H:Me) | 40 - 45 | 15 - 20 | 0,15 - 0,20 |
| SICAN (a-C:H:Si) | 30 - 35 | 10 - 15 | 0,07 - 0,15 |
| SICON®(a-C:H:Si:O) | 22 - 26 | 7 - 9 | 0,50 - 0,60 |
| F-DLC (a-C:H:F) | 20 - 22 | 2 | - |
| CrN | 30 - 75 | 18 - 20 | 0,50 - 0,80 |
| PTFE (Teflon®) | 18,5 | 0,3 | 0,10 |
| Stahl 100Cr6 | > 1000 | 8 - 9 | 0,70 - 0,90 |

Wie die Aufstellung zeigt, zeichnet sich Teflon durch eine besonders geringe Oberflächenenergie aus, welche zu einer inerten Oberfläche führt. Ein Problem der Teflonbeschichtungen ist jedoch einerseits eine fehlende Resistenz gegenüber höheren Temperaturen (über 260 °C) und andererseits eine fehlende Beständigkeit gegenüber Abrieb.

Im Falle des Einsatzes von Teflon für künstliche Acetabulumpfannen kam es durch Abrasionen zu Partikel- und somit zu Granulombildungen, die eine Prothesenentfernung zur Folge hatten. Auch wurden Fremdkörperreaktionen und die Lockerung der Implantate beim Einsatz von Teflon- Proplast-Implantaten im Temporomandibulargelenk beschrieben (Ga-Young Park Osteoblastenspezifische Biokompatibilität und mechanische Eigenschaften von etablierten und nicht etablierten Polymeren im Hinblick auf den Einsatz als Schädelimplantat, Dissertation Ruhr Universität Bochum 2012 mWN., veröffentlicht unter http://www-brs.ub.ruhr-unibochum.de/netahtml/HSS/Diss/ParkGaYoung/diss.pdf)

Aufgrund bestehender Nachteile besteht im Stand der Technik ein hohes Bedürfnis neue Beschichtungen zu finden, die bei höheren Temperaturen einsatzfähig sind und die eine hohe Abriebfestigkeit besitzen.

Bei bisherigen dafür in Frage kommenden Beschichtungen wie den in obiger Tabelle aufgeführten werden die Beschichtungen durch Adhäsion auf die Oberfläche aufgetragen, wodurch die Abriebsfestigkeit vermindert sein kann. Auch können zu Teflon alternative Beschichtungen die niedrigen Oberflächenenergiewerte von 18,5 mN/m nicht erreichen.

### Aufgabe der Erfindung

Die Aufgabe der Erfindung war es, ein Verfahren zur Inertisierung von Oberflächen und chemischen Verbindungen bereitzustellen, welches die Nachteile des Standes der Technik beseitigt. Insbesondere war es die Aufgabe der Erfindung, ein Verfahren zur Inertisierung von Keramik- Metall- oder Kunststoffoberflächen sowie inertisierte Keramik-, Metall- oder Kunststoffoberflächen bereitzustellen, welche sich durch eine geringe Oberflächenenergie und hohe Abriebsfestigkeit sowie eine hohe Beständigkeit gegenüber eines chemischen oder enzymatischen Abbaus auszeichnen.

### Zusammenfassung der Erfindung

Erfindungsgemäß wird die Aufgabe durch die unabhängigen Ansprüche gelöst. Die abhängigen Patentansprüche stellen bevorzugte Ausführungsformen Erfindung dar.

In einer bevorzugten Ausführungsform betrifft die Erfindung ein Verfahren zur Inertisierung von Materialoberflächen umfassend folgende Schritte
a) Bereitstellung einer Materialoberfläche
b) Bereitstellung einer funktionalisierten perfluorierten Verbindung
c) Reaktion der funktionalisierten perfluorierten Verbindung mit der Materialoberfläche zur Ausbildung einer kovalenten Bindung, einer lonenbeziehung oder einer Metallbindung zwischen der perfluorierten Verbindung und der Materialoberfläche.

Im Sinne der Erfindung wird unter der Inertisierung von Materialoberflächen bevorzugt eine Modifizierung der Oberflächen verstanden, welche dazu führt, dass die modifizierten Oberflächen eine geringe Haftung oder Benetzbarkeit durch Fremdmaterialien erfahren. Bevorzugt entspricht die beschriebene Inertisierung der Materialoberflächen einer Verringerung der Oberflächenenergie, die durch die Bindung der perfluorierten Verbindungen an die Materialoberflächen bewirkt wird. Dadurch werden Wechselwirkungen mit anderen Molekülen (z.B. Proteinen, Zellen oder anderen Substanzen) auf ein Minimum reduziert und es entstehen hoch inertisierte Oberflächen.

Zum Zwecke der Inertisierung ist es bevorzugt, dass eine hinreichende Flächendichte der perfluorierten Verbindungen erreicht wird, sodass die Materialoberfläche durch die perfluorierten Verbindungen von äußeren Molekülen abgeschirmt wird. Bei der Anbindung von perfluorierten Verbindungen an Kunststoffen bei Derivatisierungen für Analyseverfahren ist dies nicht der Fall.

Auf molekularer Ebene kann diese teilweise durch die Beeinflussung der perfluorierten Verbindungen auf elektrische Felder oberhalb der Materialoberfläche erklärt werden (L. Mayrhofer J. Am. Chem. Soc., 2016, 138 (12), pp 4018-4028). Durch eine hinreichende Flächendichte von perfluorierten Verbindung kommt es zu einem schnellen Abfall des elektrischen Feldes oberhalb der Materialoberfläche. Das führt dazu, dass andere Moleküle (z.B. Thrombozyten oder Schmutz), die bei nicht perfluorierten Flächen im elektrischen Feld oberhalb der Oberfläche gefangen werden, bei perfluorierten Flächen kein elektrisches Feld mehr vorfinden, in dem sie sich verfangen könnten. In bevorzugten Ausführungsformen werden mehr als 50%, bevorzugt mehr als 60%, 70%, 80% oder 90% der Materialoberfläche von den perfluorierten Verbindungen abgedeckt.

Darüber hinaus stellt die Ausbildung einer kovalenten Bindung ein besonders stabiles Anbinden der perfluorierten Verbindungen sicher, sodass die inerten Oberflächeneigenschaften auch über einen langen Zeitraum aufrechterhalten werden.

In einer bevorzugten Ausführungsform der Erfindung ist die Materialoberfläche eine Metall-, Keramik- oder Kunststoffoberfläche. Es können aber auch andere Materialoberflächen, wie beispielsweise Glasoberflächen mittels der beschriebenen Verfahren inertisiert werden.

Es ist besonders bevorzugt, dass durch das Verfahren eine unmittelbare kovalente Bindung zwischen der Materialoberfläche und den perfluorierten Verbindungen erzeugt wird. In anderen Ausführungsformen der Erfindung kann es aber auch bevorzugt sein, dass zunächst auf der Materialoberfläche eine Zwischenschicht aufgebracht wird, an welche die perfluorierten Verbindungen kovalent gebunden werden.

Zur Vermittlung einer kovalenten Bindung sind die vorgesehenen perforierten Verbindungen funktionalisiert. Im Sinne der Erfindung bezeichnet eine funktionalisierte perfuorierte Verbindungen bevorzugt eine perfluorierte Verbindung, welche mindestens eine funktionelle Gruppe aufweist. Die funktionelle Gruppe vermittelt dabei bevorzugt die kovalente Bindung mit Atomen der Materialoberfläche, bevorzugt der Keramik-, Metall- oder Kunststoffoberfläche bzw. einer Zwischenschicht. In einer weiteren Anbindungsvariante zwischen Keramik- oder Metalloberfläche und den perfluorierten Verbindungen sind starke lonenbeziehungen oder Metallbindungen möglich.

Kovalente Bindungen dienen in der Chemie zur Bildung von Molekülen. Dabei überlappen die Atomhüllen mindestens zweier Atome, sodass die anziehenden elektromagnetischen Kräfte zwischen Elektronen und Atomkernen zum Tragen kommen. Diese stehen bei einer Bindung im Gleichgewicht mit abstoßenden Coulombkräften zwischen je zwei Atomkernen oder zwischen Elektronen. Liegt eine Elektronegativitätsdifferenz (Elektronegativität als Maß für die Fähigkeit eines Atoms Elektronen anzuziehen) zwischen den Bindungspartnern vor (bis ca. 1,5), spricht man bevorzugt von polaren Bindungen. Bei sehr hohen Elektronegativitätsdifferenzen (ab ca. 1,5) kommt der Grenzfall einer kovalenten Bindung zum Tragen, die ionische Bindung. Diese basiert im Wesentlichen auf der elektrostatischen Anziehung zwischen ungeichnamig geladenen Ionen.

Im Falle der im Erfindungsgegenstand genannten Keramiken liegt häufig eine Elektronegativitätsdifferenz von >1,5 vor. Diese weist aber nicht die gleichen Eigenschaften hinsichtlich ihrer Löslichkeit in Wasser auf, wie das für Stoffe (vor allem Salze) mit ähnlicher Differenz zutrifft. Salze (die zumeist in lonengittern vorliegen) wie z.B. Natriumchlorid sind in Wasser leicht hydratisierbar, da dabei die Gitterenergie des lonengitters des Salzes überwunden wird. Bei Keramiken wird dieses Phänomen verhindert, indem der Herstellungsprozess entsprechend angepasst wird. Z.B. wird durch den Brennvorgang und/oder durch Glasuren ein späteres Auflösen in Wasser verhindert. Da Keramiken durch die Herstellungsverfahren inertisiert werden gegenüber den meisten Lösemitteln, ist die Aufbringung anderer Stoffe auf deren Oberfläche nicht ohne weiteres möglich, weshalb geeignete funktionelle Gruppen, besonders bevorzugt beispielsweise der Reaktionstyp der nucleophilen Substitution gewählt werden kann, um die Oberfläche chemisch zu verändern.

Die funktionellen Gruppen der funktionalisierten perfluorierten Verbindungen erlaubten somit bevorzugt eine Addition, Substitution, Veresterung, Veresterung, Kondensation oder andersartige Verknüpfungsreaktionen um eine kovalente Verbindung oder eine lonenbeziehung oder eine Metallbindung herzustellen.

Dem Fachmann sind verschiedene Funktionalitäten oder funktionelle Gruppen bekannt mit denen er kovalente Verknüpfungsreaktionen herzustellen vermag.

Bevorzugte funktionelle Gruppen oder Funktionalitäten sind ausgewählt sind aus der Gruppe enthaltend Halogenalkane, Hydroxyl-, Ether-, Amino-, Sulhydryl-, Aldehyd-Keto-, Carboxyl-, Ester- und Säureamidgruppen, Gruppen mit Radikalen oder Ionen und Moleküle aus den Stoffgruppen der Carbonsäuren, Peroxycarbonsäuren, Thiocarbonsäuren, Sulfonsäuren, Sulfinsäuren, Sulfensäuren, Sulfoxiden, Carbonsäuresalzen, Sulfonsäuresalzen, Sulfinsäuresalzen, Sulfensäuresalzen, Carbonsäureanhydriden, Carbonsäureestern, Sulfonsäureestern, Carbonsäurehalogeniden, Sulfonsäurehalogeniden, Carbonsäureamiden, Sulfonsäureamiden, Carbonsäurehydraziden, Nitrilen, Aldehyden, Thioaldehyden, Ketonen, Thioketonen, Oximen, Alkoholen, Phenolen, Thiolen, Aminen, Iminen, Hydrazinen, Ethern, Estern, Thioethern, Thioestern, Halogenwasserstoffen, Nitroverbindungen, Nitrosoverbindungen, Azoverbindungen, Diazoverbindungen, Diazoniumsalzen, Isocyanaten, Cyanaten, Isocyaniden, Thiocyanaten, Isothiocyanaten, Hydroperoxiden, Peroxiden, 2'-Carbamate, Ether, Säureamide und Thioether oder Verbindungen, die aufgrund ihrer Mehrfachbindung reaktiv sein können.

Besonders bevorzugt sind Heteroatome wie Br, I, Cl, H, Si, N, O, S, P, Hydroxyl-, Amino-, Carboxylgruppen, Halogenalkane, Carbonsäureamiden, Alkoholen, Hydrazinen, Isocyanaten, Thiocyanaten und Säureamide.

In einer bevorzugten Ausführungsform werden funktionalisierte perfluorierte Verbindungen genutzt, die zur nukleophilen Substitution geeignet sind. Bevorzugt handelt sich bei den funktionellen Gruppen um nukleophile Abgangsgruppen, welche eine nukleophile Substitution erlauben.

Dabei meint nukleophile Substitution den Austausch einer Gruppe oder eines Atoms (im Weiteren als Abgangsgruppe bezeichnet) durch eine neue Gruppe oder ein neues Atom. Die Abgangsgruppe sollte dafür eine geringere Nukleophilie aufweisen als das angreifende Nukleophil. Hier meint Nukleophil ein Molekül, das entweder eine negative Ladung (Elektronenpaardonator) oder Partialladung trägt oder dem klassischen Konzept einer Lewis-Base genügt. Für die Bestimmung der Nukleophilie wird die Elektronegativität und der sich daraus ergebende Dipol oder Monopol betrachtet. Dabei gilt, je größer die Elektronegativität, desto stärker das Nukleophil.

Bei der nukleophilen Substitution greift das Nukleophil das organisch chemische Molekül an jenem C-Atom an, an dem sich auch die Abgangsgruppe befindet. Dabei wird hier auf zwei Reaktionstypen zurückgegriffen, die nukleophile Substitution 1. Ordnung (S_{N}1) und die nukleophile Substitution 2. Ordnung(S_{N}2). Diese unterscheiden sich wie folgt:

### Allgemeiner Mechanismus S_{N}1:

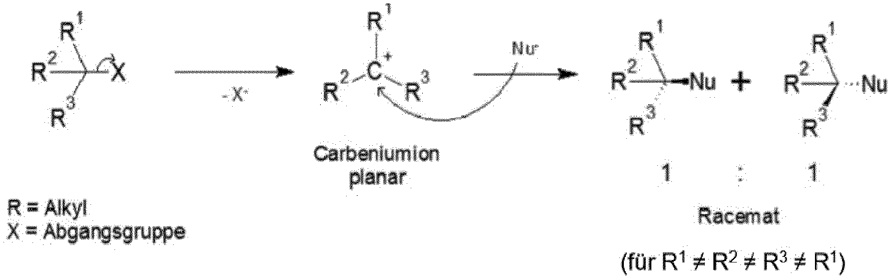

### Allgemeiner Mechanismus S_{N}2:

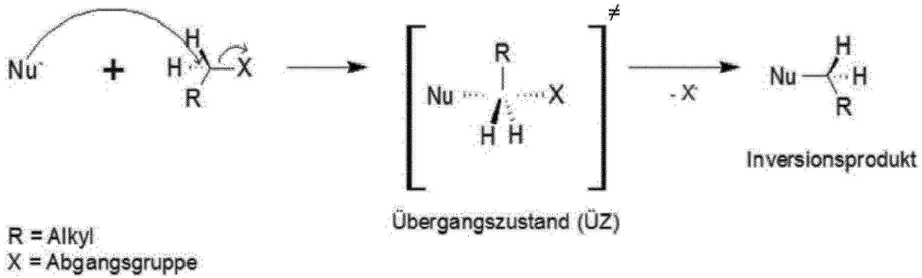

Bei den hier verwendeten perfluorierten Kohlenwasserstoffen wird die funktionelle Gruppe als Nukleophil betrachtet, da diese, wie bei der OH-Gruppe, entweder bereits durch einzelne stark elektronegative Atome als Nukleophil geeignet sind, oder, wie im Falle der COOH-Gruppe oder SO₃H-Gruppe, leicht deprotoniert werden können und so eine negative Ladung erhalten.

In einer weiteren Ausführungsform werden die funktionalisierten perfluorierten Verbindungen über radikalische Substitution, radikalische Addition oder andere radikalische Reaktionen an die Oberfläche gebunden.

In einer weiteren Ausführungsform werden die funktionalisierten perfluorierten Verbindungen über UV-Aktivierung oder über die Aktivierung mittels anderer Energiequellen an die Oberfläche gebunden.

In einer bevorzugten Ausführungsform der Erfindung ist das Verfahren somit dadurch gekennzeichnet, dass die funktionalisierten perfluorierten Verbindungen eine perfluorierte Verbindung mit mindestens einer funktionellen Gruppe ist, die mindestens eine Doppelbindung umfasst und durch UV-Quervernetzung angebunden werden kann, eine perfluorierte Verbindung mit mindestens einer funktionellen Gruppe ist, welche an Radikale oder Protonen angebunden werden kann, welche bevorzugt nach einer Plasma- oder Koronabehandlung der Materialoberfläche entstehen und/oder eine perfluorierte Verbindung mit mindestens einer funktionellen Gruppe ist, welche für die Cycloaddition geeignet ist.

Ein Fachmann kennt verschiedene Formen funktioneller Gruppen, welche geeignet sind für eine UV Quervernetzung, zur Anbindung an Radikale oder Protonen oder eine Anbindung mittels Cycloaddition. Auch sind verschiedene nukleophile Substitutionen und geeignete nukleophile Abgangsgruppen dem Fachmann bekannt.

In einer bevorzugten Ausführungsform ist die funktionalisierte perfluorierte Verbindung eine perfluorierte Verbindung mit mindestens einer funktionellen Gruppe, wobei die funktionelle Gruppe bevorzugt eine nukleophile Abgangsgruppe ist, besonders bevorzugt ausgewählt aus einer Gruppe umfassend Br, I, Cl, H, N, O, S, P, Hydroxyl-, Amino-, Carboxylgruppen,Carbonsäuren, Thiocarbonsäuren, Sulfonsäuren, Sulfinsäuren, Carbonsäurehalogeniden, Sulfonsäurehalogeniden, Säureamide, Carbonsäureamiden, Alkohole, Sulfonsäureamiden, , Phenolen, Hydrazinen, Thiolen, Aminen, Iminen, Hydrazinen, Isocyanaten, Thiocyanaten, Isothiocyanaten.

Mit den vorgenannten bevorzugten funktionellen Gruppen, insbesondere mit den bevorzugten nukleophilen Abgangsgruppen, werden zuverlässig und wiederholbar stabile Anbindungen realisiert. Die derart modifizierten Oberflächen zeichnen sich zudem durch eine besonders hohe Abriebsfestigkeit aus.

Die funktionalisierten perfluorierten Verbindungen umfassen bevorzugt einen Molekülrest bestehend aus einer perfluorierten Verbindung sowie eine funktionalisierte Gruppe zur Vermittlung einer kovalenten Bindungen zwischen der perfluorierten Verbindung und der Materialoberfläche, bevorzugt der Keramik-, Metall- oder Kunststoffoberfläche.

Im Sinne der Erfindung bezeichnen den perfluorierten Verbindungen bevorzugt geradlinige oder verzweigte acyclische oder cyclische, polycyclische oder heterocyclische aliphatische Alkane, Alkene, Alkine, aromatische Verbindungen oder Kombinationen aus diesen oder Silane, bei denen alle H-Atome durch F-Atome ersetzt sind, die optional zusätzlich nicht-fluorierte oder teilfluorierte Substituenten mit einer oder mehreren funktionellen Gruppen oder Heteroatomen, insbesondere Br, I, Cl, H, AI, N, O, S, P, oder diese in Verbindung mit einer oder mehreren weiteren funktionellen Gruppen besitzen.

In einer bevorzugten Ausführungsform der Erfindung sind die funktionalisierten perfluorierten Verbindungen funktionalisierte Perfluorcarbone (PFC).

Moleküle ausgewählt aus der Gruppe der Perfluorocarbone (PFC), bezeichnen bevorzugt geradlinige oder verzweigte acyclische oder cyclische, polycyclische oder heterocyclische aliphatische Alkane, Alkene, Alkine, aromatische Verbindungen oder Kombinationen aus diesen Verbindungen sein können, bei denen alle H-Atome durch F-Atome ersetzt sind, die optional zusätzlich mindestens einen nicht-fluorierten oder teilfluorierten Substituenten in Form von einer oder mehreren funktionellen Gruppen, aliphatischen Ketten oder Heteroatomen, insbesondere Br, I, Cl, H, Si, N, O, S, P, besitzen oder diese in Verbindung mit einer oder mehreren weiteren funktionellen Gruppen stehen.

Es ist besonders bevorzugt, dass die Perfluorocarbone (PFC) ausgewählt sind aus der Gruppe enthaltend C₁-C₁₀₀, bevorzugt C₁-C₅₀, insbesondere bevorzugt C₁-C₃₀, ganz bevorzugt C₁-C₂₀, höchst bevorzugt C₁-C₂₀ Alkane, Alkene oder Alkine, die linear, verzweigt, cyclisch, polycyclisch oder heterocyclisch sein können, C₆-C₅₀, bevorzugt C₆-C₃₀, insbesondere bevorzugt C₁-C₂₀ aromatische oder heteroaromatische Systeme können ebenfalls bevorzugt sein. In den vorgenannten Verbindungen sind die C-Atome bevorzugt weitestgehend, ganz bevorzugt vollständig,perfluoriert.

In einer bevorzugten Ausführungsform umfassen die funktionalisierten Perfluorocarbone zwischen 1 und 50, bevorzugt zwischen 1 und 20, besonders bevorzugt zwischen 1 und 10 ganz besonders bevorzugt zwischen 2 und 10 perfluorierte C-Atome. Bevorzugt sind die C-Atome des Molekülrestes der Perfluorocarbone, also dem Bestandteil ohne funktionelle Gruppe, vollständig fluoriert.Typischerweise können im Rahmen der vorliegenden Erfindung Molekülreste ausgewählt aus der PFC-Gruppe enthaltend-(CₙF₍₂ₙ₊₂₎₋₁) mit n ≥ 1, bevorzugt n = 1-20, wie z.B.-CF₃, -C₂F₅, -C₃F₇, -C₄F₉, -C₅F₁₁ usw., -(CₙF₂ₙ₋₁) mit n ≥ 2, bevorzugt n = 2-20 wie z.B. -C₂F₃, -C₃F₅, -C₄F₇ usw., -(-CₙF₍₂ₙ₋₂₎₋₁) mit n ≥ 2, bevorzugt n = 2-20, wie z.B. -C₂F, -C₃F₃, -C₄F₅, -C₅F₇ usw. verwendet werden.

In einer besonders bevorzugten Ausführungsform der Erfindung umfasst die funktionalisierte perfluorierte Verbindung eine perfluorierte Verbindung und eine funktionelle Gruppe, wobei die perfluorierte Verbindung höchstens 20, ganz besonders bevorzugt höchsten 10, vollständig fluorierte Atome umfasst. Ganz besonders bevorzugte perfluorierte Verbindungen als Molekülrest sind somit PFC-Gruppen mit der Summenformel -(CₙF₂ₙ₊₁) mit n ≥ 1, bevorzugt n = 1-20, bevorzugt n = 2-20, höchst bevorzugt n=2-10. Weitere ganz bevorzugte perfluorierte Verbindungen als Molekülrest sind somit Gruppen mit Summenformel SiₙF₂ₙ₊₁ mit n ≥ 1, bevorzugt n = 1-20, bevorzugt n = 2-20, höchst bevorzugt n=2-10. Diese perfluorierte Verbindungen werden bevorzugt auch als kurzkettige Molekülreste bezeichnet.

Im Stand der Technik ist es bevorzugt zur Inertisierung von Oberflächen möglichst langkettige Perfluorocarbone als Makromoleküle mit mehr als 20 perfluorierten C-Atomen zu verwenden. Die Erfinder haben jedoch erkannt, dass im Zusammenhang mit funktionalisierten Perfluorcarbonen, welche kovalent an die Oberflächen gebunden werden, überraschenderweise kurzkettige Moleküle bessere Ergebnisse liefern. Hierdurch können besonders stabile und langlebige Beschichtungen erreicht werden, welche dennoch ausgezeichnete inerte Eigenschaften aufweisen. Insbesondere durch die vorgenannten Verbindungen war es überraschender Weise möglich eine kovalente besonders stabile Inertisierung von Materialoberflächen zu erreichen, welche zu einer Reduktion der Oberflächenenergien auf einen Bereich von 20 mN/m oder weniger führt. Die bevorzugten funktionalisierten Verbindungen vereinen somit auf besondere Weise die vorteilhaften inerten Eigenschaften von Teflon mit einer hohen chemischen wie mechanischen Beständigkeit

Bevorzugt werden funktionalisierte Perfluorcarbone genutzt, die zur nukleophilen Substitution geeignet sind.

In einer bevorzugten Ausführungsform der Erfindung basieren die funktionalisierten perfluorierte Moleküle auf einer der folgenden Substanzen:
- F(CF₂)ₙX, wobei n= 1-50, bevorzugt n = 1-10, und X = Br, I, Cl, H oder X= Si, N, O, S, P, in Verbindung mit einer funktionellen Gruppe, insbesondere C₈F₁₇I, C₈ F₁₇Br;
- F(CF₂)ₙ-(CH₂)ₘX, wobei n = 1-50, bevorzugt n = 1-10, m = 1-26, bevorzugt m = 1 - 6 und X = Si, N, O, S, P, Br, I, H;
- F(CF₂)n-O_{b}-CH=CH₂ mit n = 1-50, bevorzugt n = 1-10, und b = 0 oder 1, bevorzugt b = 0;
- C₆F₁₃CH₂CH₂Mgl, (C₆F₁₃CH₂CH₂)₃SnPh, (C₆F₁₃CH₂CH₂)₃SnBr, (C₆F₁₃CH₂CH₂)₃SnH;
- C₂F₅I, C₃F₇Br, C₄F₉I, C₅F₁₁Br, C₆F₁₃Br, C₈F₁₅Br, C₁₀F₁₇I,
- C₄F₉CH=CHC₄F₉, C₈F₁₆Cₗ₂, C₁₀F₁₉N, C₆F₁₉Br, C₉F₂₁N, C₁₀F₂₁Br, C₁₁F₂₂N₂O₂, C₆F₁₃CH=CHC₆F₁₃, C₁₂F₂₇N, C₁₆F₂₅Br;
- C₈F₁₇I, C₈ F₁₇Br

Besonders bevorzugt sind auch Metallorganyle (z.B. Lithiumorganyl) als funktionalisierten perfluorierte Moleküle, also perfluorierte Kohlenstoffatomverbindungen, wobei mindestens an einem Kohlenstoffatom ein Metall angebracht vorliegt

Die bevorzugten Modifizierungen führen zu Oberflächen mit äußerst geringen Oberflächenenergien und den sich daraus ergebenden Eigenschaften wie chemische Beständigkeit, sehr geringe Oberflächenreibung, Verhinderung der Anlagerung von hydrophilen oder lipophilen Substanzen oder von Zellen.

Weiterhin sind die Modifizierungen resistent gegen enzymatischen Abbau (beispielsweise durch Hydrolasen oder Proteasen), wodurch sie sich insbesondere für medizinische Anwendungen eignen. Zudem sind die Modifizierungen biokompatibel und medizinisch nutzbar, da bereits ähnliche Moleküle in medizinischen Anwendungen für andere Funktionen genutzt werden, beispielsweise als Kontrastmittel oder als Blutersatzstoff.

Weitere bevorzugte Ausgangssubstanzen der funktionalisierten perfluorierten Verbindungen aus der Gruppe der PFCs, welche im Rahmen der Erfindung verwendet werden können sind:
- perfluorierte Cholesteryl- und Adamantylverbindungen, perfluorierte cis-Eicosenoic, perfluorierte aromatische Verbindungen, perfluorierte Pyrene, perfluorierte Glyceride; und 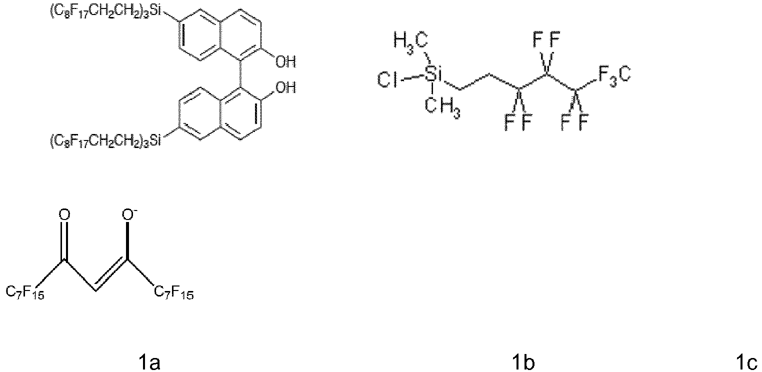 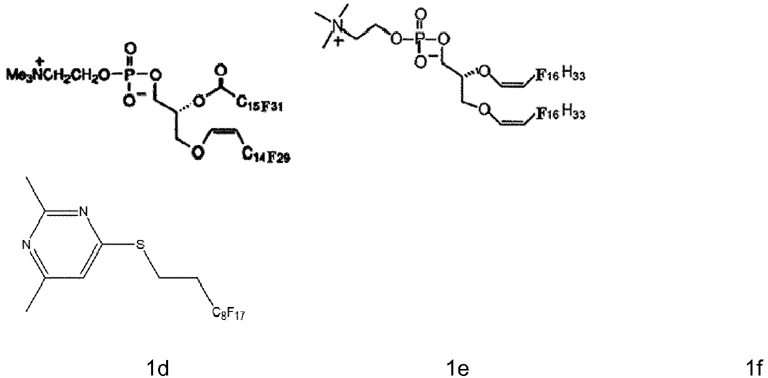 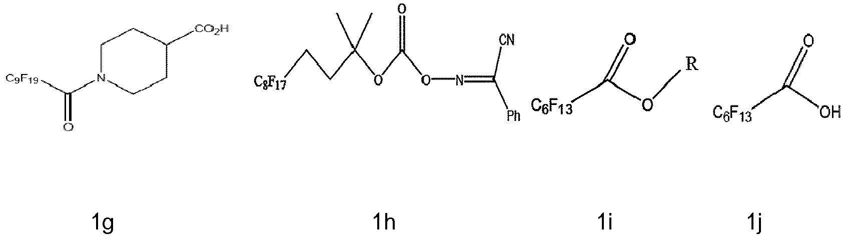 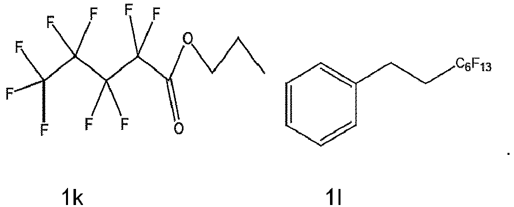
- zur Verknüpfung in Form einer lonenbindung können z.B. folgende PFC's verwendet werden: 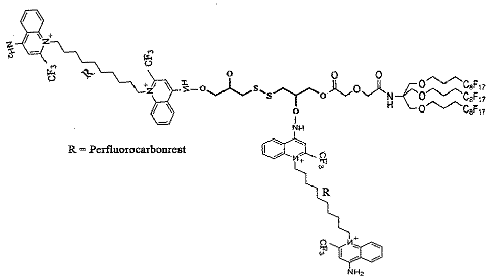 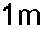 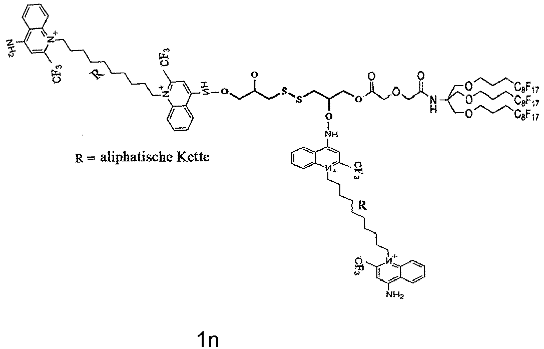 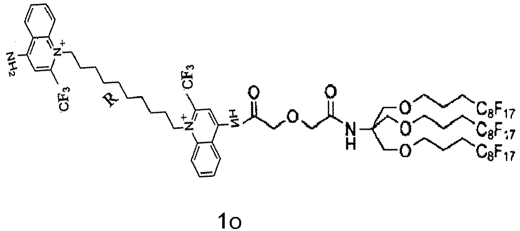 mit R = Perfluorocarbonrest oder aliphatische Kette;

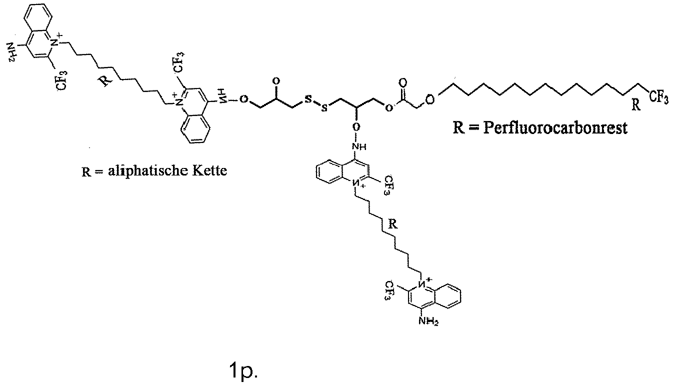

In einer bevorzugten Ausführungsform der Erfindung sind die perfluorierten Verbindungen Perfluorsiliziumverbindungen.

In dieser Ausführungsform stammt der perfluorierte Teil oder Molekülrest aus der Gruppe der Perfluorsiliziumverbindungen enthaltend geradlinige oder verzweigte acyclische oder cyclische, polycyclische oder heterocyclische aliphatische Silane, bei denen alle H-Atome durch F-Atome ersetzt sind, die optional zusätzlich nicht-fluorierte oder teilfluorierte Substituenten mit einer oder mehreren funktionellen Gruppen oder Heteroatomen, insbesondere Br, I, Cl, H, AI, N, O, S, P oder diese in Verbindung mit einer oder mehreren weiteren funktionellen Gruppen besitzen.

Bevorzugt werden Perfluorsiliziumverbindungen aus der Gruppe enthaltend Si₁-Si₁₀₀, bevorzugt Si₁-Si₅₀, insbesondere bevorzugt Si₁-Si₃₀, ganz bevorzugt Si₁-Si₂₀, höchst bevorzugt Si₁-Si₂₀ perfluorierte Siliziumverbindungen verwendet. Die Perfluorsiliziumverbindungen liegen ebenfalls wie im Falle der PFC's mit geeigneten Substituenten funktionalisiert vor. D.h. die funktionalisierten Perfluorsiliziumverbindungen weisen einen perfluorierten Teil oder Molekülrest, wie obig beschrieben auf, und zusätzlich mindestens eine funktionelle Gruppe zur Vermittlung einer kovalenten Bindung.

In einer bevorzugten Ausführungsform umfassen die funktionalisierten Perfluorsiliziumverbindungen zwischen 1 und 50, bevorzugt zwischen 1 und 20, besonders bevorzugt zwischen 1 und 10 ganz besonders bevorzugt 2 und 10 perfluorierte Si-Atome. Bevorzugt sind die Si-Atome des Molekülrestes also dem Bestandteil ohne funktionelle Gruppe, vollständig fluoriert.

Weitere bevorzugte funktionalisierte Perfluorsiliziumverbindungen können aus der folgenden Gruppe ausgewählt sein:
- Siloxane der allgemeinen Formel -[-SiR₂-O-]ₙ- mit n≥1 und R = perfluorierte Carbone oder F,
- Siloxanole der allgemeinen Formel HO-A-[-SiR₃R₄-O-]-SiR₃R₄R₅ mit R_{3,4,5} = F oder -F(CF₂)ₙ, mit n = 1 - 10 und A = Alkylkette
- perfluorierte Silicate, Kieselsäuren, Natriumsilicate, Polysilazane, Silicide, Siliciumtetrahalogenide, Silikone, Silikonöle, Zeolithe, Zirkonsilikate.
- Silane der allgemeinen Formel X-CH₂-Si(O_{R})₃, wobei X eine für die nukleophile Substitution geeignete Abgangsgruppe mit X = Cl, Br, I, H, OH oder funktionelle Gruppen umfassend Amino-, Vinyl-, Carbamato-, Glycidoxy-, Methylalkoxy-, Phenyl- oder Acetoxygruppen und R = F oder -F(CF₂)ₙ mit n = 1 - 10 ist,
- Silane der allgemeinen Formel X-Si (CF₃)₃ oder X-Si(R)₃, wobei X eine für die nukleophile Substitution geeignete Abgangsgruppe mit X = Cl, Br, I, H, OH oder funktionellen Gruppen wie Amino-, Vinyl-, Carbamato-, Glycidoxy-, Methylalkoxy-, Phenyl- oder Acetoxygruppen und und R = F oder R = F(CF₂)ₙ mit n = 1 - 10 ist.

In einer weiteren Ausführungsform ist die perfluorierte Verbindung ausgewählt aus der Gruppe weiterer perfluorierter Verbindungen, wobei es auf Verbindungen basiert, ausgewählt aus NF3, N2F4, SNF3, CF3SN, SF4, SF6, perfluorierte Stickstoff-Schwefelverbindungen.

In einer weiteren Ausführungsform besteht der perfluorierte Molekülteil aus Fluor-Phosphor-Verbindungen oder Fluor-Aluminium-Verbindungen.

Auch in diesen Fällen ist es besonders bevorzugt, dass die perfluorierte Verbindung durch kurzkettige Moleküle kennzeichnet ist.

In einer bevorzugten Ausführungsform umfassen die funktionalisierten perfluorierten Verbindungen zwischen 1 und 50, bevorzugt zwischen 1 und 20, besonders bevorzugt zwischen 1 und 10 ganz besonders bevorzugt 2 und 10 perfluorierte Atome, wobei die Atome bevorzugt ausgewählt sind auch der Gruppe C, Si, S oder N oder Kombinationen dieser wie bspw. SN (Stickstoff-Schwefel).

Bevorzugt sind die Atome des Bestandteils der perfluorierten Verbindung ohne Funktionalität, vollständig fluoriert. D.h. sämtliche Wasserstoffanbindungen an die Atome wurden durch Fluor substituiert.

Es kann auch bevorzugt sein, dass die perfluorierten Verbindung zwei und mehr perfluorierte Moleküle ausgewählt aus der Gruppe der Perfluorocarbone (PFC), Perfluorsiliziumverbindungen und weiterer perfluorierter Verbindungen enthalten.

Typischerweise können die oben beschriebenen funktionalisierten perfluorierten Verbindungen zu mehreren Einheiten zusammengefasst werden. Als Beispiele sei auf die folgenden Moleküle hingewiesen:

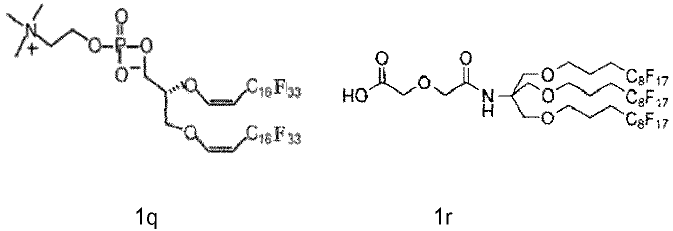

Die Anbindung dieser Moleküle an die weiteren in der erfindungsgemäßen Verbindung enthaltenen Moleküle kann über NH₂-Gruppen oder OH-Gruppen oder andere geeignete Gruppen erfolgen.

Vorteilhafterweise lassen sich durch die offenbarten funktionalisierten perfluorierten Verbindungen eine Vielzahl verschiedener Keramikoberflächen inertisieren.

Im Sinne der Erfindung umfasst der Begriff Keramik keramische Werkstoffe, welche anorganisch, nicht-metallisch und polykristallin sind.

Die Keramik kann beispielsweise ausgewählt sein aus einer Gruppe enthaltend Aluminiumoxid, Zirkoniumoxid, Aluminiumtitanat, Siliziumkarbid, Siliziumnitrid oder Aluminiumnitrid oder andere nichtmetallische oder metallische Stoffe oder Dispersionskeramiken und Piezokeramiken, Porzellan, Steinzeug, Stealit, Glaskeramik, Cordlerit, Titanoxid, Yttriumoxid, Berylliumoxid, Magnesiumoxid, Uranoxid, Titanate. Bleititanalzirkonat, Ferrite, Kohlenstoff, Nitride, Carbide, Boride, Silicide (Silicium, Bor, Titan, Molybdän).

Besonders bevorzugt sind Hochleistungskeramiken oder technische Keramiken, welche auf technische Anwendungen hin optimiert wurden. Beispielsweise können diese besondere Härten, Leitfähigkeiten oder Temperaturverhalten etc. aufweisen, um den jeweiligen Anforderungen für die gewünschten Anwendung zu genügen.

Werkstoffe für die Hochleistungskeramiken wie z.B. aus Aluminiumoxid, Zirkoniumoxid, Aluminiumtitanat, Siliziumkarbid, Siliziumnitrid oder Aluminiumnitrid oder andere nichtmetallische oder metallische Stoffe oder Dispersionskeramiken und Piezokeramiken gewinnen seit der Weiterentwicklung des 3D-Drucks immer mehr an Bedeutung. Sie finden wachsende Einsatzmöglichkeiten unter anderem im medizinischen Bereich (Implantate, Zahntechnik) und in Bereichen hoher mechanischer Beanspruchung sowie in Zirkoniumoxid stabilisierten Substraten. Die Eigenschaft einiger dieser Stoffe, besonders leitfähig oder magnetisch bzw. diamagnetisch zu sein, bergen weitere interessante Anwendungsgebiete.

In einer bevorzugten Ausführungsform der Erfindung wird die Keramikoberfläche von einer Keramik gebildet wird ausgewählt ist aus einer Gruppe enthaltend Aluminiumoxid, Zirkoniumoxid, Aluminiumtitanat, Siliziumkarbid, Siliziumnitrid oder Aluminiumnitrid oder andere nichtmetallische oder metallische Stoffe oder Dispersionskeramiken und Piezokeramiken, Porzellan, Steinzeug, Stealit, Glaskeramik, Cordlerit, Titanoxid, Yttriumoxid, Berylliumoxid, Magnesiumoxid, Uranoxid, Titanate. Bleititanalzirkonat, Ferrite, Kohlenstoff, Nitride, Carbide, Boride, Silicide (Silicium, Bor, Titan, Molybdän), wobei Hochleistungskeramiken insbesondere Aluminiumoxid, Zirkoniumoxid, Titanoxid, Aluminiumtitanat, Siliziumkarbid, Siliziumnitrid oder Aluminiumnitrid besonders bevorzugt sind.

Insbesondere für die letztgenannten Hochleistungskeramiken, ganz bevorzugt Aluminiumoxid, Zirkoniumoxid, Titanoxid, Aluminiumtitanat, Siliziumkarbid, Siliziumnitrid oder Aluminiumnitrid konnten ausgezeichnete Ergebnisse erzielt werden. Nicht nur lassen sich die funktionalisierten perfluorierten Verbindungen an diesen auf einfache Weise kovalent binden und stabilisieren, sondern durch die Verbindungen wird auch eine besonders homogene Abdeckung und Inertisierung erreicht. Überraschenderweise erhalten die funktionalisierten perfluorierten Verbindungen, insbesondere im Falle kurzkettiger perfluorierten Molekülreste, die sonstigen vorteilhaften Eigenschaften welche die Hochleistungskeramik charakterisieren, wie z.B. Härte, Korrosionsbeständigkeit, Abrieb- und Verschleißfestigkeit etc.

Beispielhaft enthalten bevorzugte Keramik-Verbindungen u.a. folgende Partikel MgO, ZrO2, B4C, S3C, Si3N4, BN, Al2TiO5, Pb(Zr, Ti)O2, BaTiO3, AIN oder Al2O3.

Vorteilhafterweise lassen sich durch die offenbarten funktionalisierten perfluorierten Verbindungen zudem ebenfalls verschiedene Metalloberflächen inertisieren.

In einer bevorzugten Ausführungsform wird die Metalloberfläche von einem Metall gebildet ausgewählt aus einer Gruppe enthaltend Titan, Aluminium, Cadmium, Chrom, Eisen, Gold, Iridium, Cobalt, Kupfer, Nickel, Palladium, Platin, Silber, Zink und Zinn sowie Legierungen, welche diese enthalten wie beispielsweise Titanlegierungen oder Edelstahl, ganz besonders bevorzugt wird die Metalloberfläche von Titan oder einer Titanlegierungen gebildet.

Die bevorzugt genannten Metalle, insbesondere Titan, lassen sich mit den funktionalisierten perfluorierten Verbindungen auf ähnliche Weise wie für die Keramiken erfolgreich beschichten.

Durch die Inertisierung mittels der beschriebenen funktionalisierten perfluorierten Verbindungen lassen sich die Metalle vielfältig einsetzen. Insbesondere für medizinische Anwendungen z.B. für Implantate mit Metallbestandteilen, kann durch die kovalente Anbindung der funktionalisierten perfluorierten Verbindungen sichergestellt werden, dass es nicht zu unerwünschten Anlagerungen biologischen Materials oder von Zellen kommt.

In einer bevorzugten Ausführungsform wird die Kunststoffoberfläche von einem Kunststoff gebildet, ausgewählt aus einer Gruppe enthaltend Polyacetale, Polyacrylate, Polyamide, Polyaryle, Cellulosen, Polyester, Polyoleofine, Polystyrole, Polyvinyacatate, Polyvinylchloride, Polyetherketone, Polyaryletherketone, Polylactide, Aminoharze, Epoxidharze, Polyetheralkohole, Butadienelastomere, Fluorocarbonelastomere, Esterelastomere, Isoprenelastomere, Olefinelastomere, Siloconelastomere, Pentene-Elastomere, Sulfidelastomere, Urethanelastomere und Vinylchloridelastomere. Für die vorgenannten Kunststoffe können es mit den beschrieben Verfahren auf einfache Weise funktionalisierten perfluorierten Verbindungen stabil angebunden werden. Die somit erhaltenen inertisierten Kunststoffoberflächen lassen sich vielfältig einsetzen und finden beispielsweise in Medizinprodukten, aber auch Alltagsprodukten Anwendungen, wobei eine Anlagerung von biologischem Material oder Schmutzpartikeln wirksam verhindert wird.

Die erfindungsgemäße Inertisierung mittels der beschriebenen funktionellen perfluorierten Verbindungen ist auf verschiedene Weise umsetzbar.

Zum einen kann eine Voraktivierung der Keramik- oder Metalloberflächen durch Säuren (z.B. mit Schwefelsäure oder Flusssäure), durch andere Agenzien, durch Laserbehandlung, durch Korona- oder Plasmabehandlung, durch Ozonbehandlung oder andere Verfahren erfolgen und anschließende Reaktion mit funktionalisierten perfluorierten Verbindungen zur kovalenten Anbindung oder zur Ionenbeziehung oder zur Metallbindung führen. Auch eine direkte Reaktion mit funktionalisierten perfluorierten Verbindungen, ohne Voraktivierung kann vorgesehen sein. Des Weiteren kann es auch bevorzugt sein, dass die Keramik- oder Metalloberflächen mit einer Zwischenschicht beschichtet werden, mit welcher die funktionalisierten perfluorierten Verbindungen reagieren.

In einer bevorzugten Ausführungsform des Verfahrens erfolgt vor der Reaktion der funktionalisierten perfluorierten Verbindungen eine Aktivierung der Materialoberfläche, wobei die Aktivierung bevorzugt durch Säure-, Laser-, Korona- , Ozon- oder Plasmabehandlung erfolgt.

Diese Ausführungsform ist besonders bevorzugt im Zusammenhang mit funktionalisierten perfluorierten Verbindungen, welche nukleophile Abgangsgruppen als funktionelle Gruppen aufweisen.

Bevorzugt führt die Aktivierung der Materialoberfläche, bevorzugt der Keramik-, Metall- oder Kunststoffoberfläche dazu, dass diese elektrophil wird bzw. elektrophile Gruppen ausbildet. Eine nukleophile Substitution mittels der bevorzugten funktionalisierten perfluorierten Verbindungen greift an den elektrophilen Gruppen an und führt zu einer kovalenten Bindung.

Dem Fachmann sind verschiedene Verfahren zur Aktivierung einer Oberfläche bekannt.

Beispielsweise kann es bevorzugt sein, zu diesem Zweck Säuren, besonders bevorzugt eine Fluorwasserstoff (Flusssäure) oder ein Schwefelsäure einzusetzen. Aber auch andere Aktivierungen beispielsweise mittels Plasma oder Laserbestrahlungen sind denkbar.

In einer bevorzugten Ausführungsform der Erfindung erfolgt für Teilbereiche der Materialoberfläche eine unterschiedliche Aktivierung. Hierdurch können unterschiedliche Inertisierungsgrade auf der Materialoberfläche kontrolliert realisiert werden. Beispielsweise kann es bevorzugt sein, bestimmte Teilbereiche zu aktivieren, während andere Teilbereich nicht aktiviert werden, sodass die perfluorierten Verbindungen nur an die aktivierten Teilbereiche binden. Auch Zwischenstufen einer unterschiedlich graduierten Aktivierung, um Beschichtungen mit unterschiedlichen Dichten der perfluorierten Verbindungen zu erzeugen, können vorgesehen sein.

Durch eine derart selektive Oberflächenbehandlung, also einer unterschiedlichen Aktivierung der Teilbereiche z.B. durch eine Laserbehandlung oder durch ein selektiv erzeugtes Druckbild und die damit verbundene Zugänglichkeit oder Nicht-Zugänglichkeit für die Reaktionen mit den funktionalisierten perfluorierten Verbindungen kann vorteilhafterweise ein definiertes Muster zwischen Bereichen unterschiedlicher Eigenschaften (z.B. hohe und sehr niedrige Oberflächenenergie) erzeugt werden.

In einer bevorzugten Ausführungsform der Erfindung erfolgt für Teilbereiche der Materialoberflächen eine unterschiedliche Aktivierung gemäß einem vorgegeben Muster, sodass eine unterschiedliche Inertisierung der Keramik- oder Metalloberfläche gemäß des vorgegebenen Musters realisiert wird.

Beispielsweise können selektiv Bereiche starker Oberflächenenergie (große Wechselwirkungen mit der Umwelt) und Bereiche sehr niedriger Oberflächenenergie (geringe Wechselwirkungen mit der Umwelt) unter physiologischen Bedingungen erzeugt werden. Oder es kann die Reibung zwischen zwei Teilbereichen von Oberflächen gezielt vermindert werden.

In einer weiteren bevorzugten Ausführungsform betrifft die Erfindung inertisierte Keramik- oder Metalloberflächen, wobei die perfluorierten Verbindungen kovalent an einer Keramik- oder Metalloberfläche gebunden vorliegen.

In einer bevorzugten Ausführungsform betrifft die Erfindung inertisierte Materialoberflächen, bevorzugt Keramik-, Metall- oder Kunststoffoberflächen herstellbar oder hergestellt durch ein erfindungsgemäßes Verfahren oder bevorzugten Ausführungsformen davon, sodass die perfluorierten Verbindungen kovalent an einer Materialoberfläche, bevorzugt einer Keramik-, Metall- oder Kunststoffoberfläche gebunden vorliegen.

Inertisierte Materialoberflächen, bevorzugt inertisierte Keramik-, Metall- oder Kunststoffoberflächen meint im Sinne der Erfindung bevorzugt eine Materialoberfläche, welche durch eine Inertisierung eine geringe Haftung oder Benetzbarkeit durch Fremdmaterialien aufweist. Die inertisierte Materialoberfläche, bevorzugt die inertisierte Keramik-, Metall- oder Kunststoffoberfläche kann auch als inerte Keramik-, Metall oder Kunststoffoberfläche bezeichnet werden.

Die inertisierten Materialoberflächen zeichnen sich durch die beschriebenen Vorteile aus. Insbesondere sind die inertisierten Materialoberflächen durch äußerst geringe Oberflächenenergien gekennzeichnet, wodurch diese eine hohe chemische Beständigkeit, geringe Oberflächenreibung und vermindertes Haftungsvermögen gegenüber Fremdmaterialien aufweisen. Insbesondere wird an den erfindungsgemäßen inertisierten Materialoberflächen eine Anlagerung von hydrophilen, lipophilen Substanzen oder Zellen wirksam verhindert, sodass sich diese insbesondere für medizinische Anwendungen eignen.

Der Fachmann erkennt, dass bevorzugte Ausführungsformen und Vorteile welche für das Verfahren zur Inertisierung von Materialoberflächen beschrieben werden, ebenso für die inertisierten Materialoberflächen gelten. Beispielsweise wurde für das Verfahren offenbart, dass bevorzugt funktionalisierte PFCs verwandt werden. Der Fachmann erkennt, dass in einer bevorzugten Ausführungsform die inertisierten Materialoberflächen durch eine kovalente Bindung von PFCs, als perfluorierte Verbindungen, gekennzeichnet sind.

In bevorzugten Ausführungsformen können bei den inertisierten Materialoberflächen, bevorzugt bei den Keramik-, Metall- oder Kunststoffoberflächen die perfluorierten Verbindungen durch ein Linker- oder Spacermolekül mit der Oberfläche des Materials, bevorzugt des Metalls, der Keramik oder des Kunststoffes verbunden sein, beispielsweise aus einer Gruppe enthaltend insbesondere Halogenalkane, Hydroxyl-, Ether-, Amino-, Sulhydryl-, Aldehyd- Keto-, Carboxyl-, Ester- und Säureamidgruppen, Gruppen mit Radikalen oder Ionen und Moleküle aus den Stoffgruppen der Carbonsäuren, Peroxycarbonsäuren, Thiocarbonsäuren, Sulfonsäuren, Sulfinsäuren, Sulfensäuren, Sulfoxiden, Carbonsäuresalzen, Sulfonsäuresalzen, Sulfinsäuresalzen, Sulfensäuresalzen, Carbonsäureanhydriden, Carbonsäureestern, Sulfonsäureestern, Carbonsäurehalogeniden, Sulfonsäurehalogeniden, Carbonsäureamiden, Sulfonsäureamiden, Carbonsäurehydraziden, Nitrilen, Aldehyden, Thioaldehyden, Ketonen, Thioketonen, Oximen, Alkoholen, Phenolen, Thiolen, Aminen, Iminen, Hydrazinen, Ethern, Estern, Thioethern, Thioestern, Halogenwasserstoffen, Nitroverbindungen, Nitrosoverbindungen, Azoverbindungen, Diazoverbindungen, Diazoniumsalzen, Isocyanaten, Cyanaten, Isocyaniden, Thiocyanaten, Isothiocyanaten, Hydroperoxiden, Peroxiden oder Verbindungen, die aufgrund ihrer Mehrfachbindung reaktiv sein können, oder funktionalisierte Perfluorkohlenwasserstoffe mit Jod-, Brom- oder Schwefelatomen, Carbamaten, Thioether- oder Disulfidgruppen, Glycerol, Succinylglycerol, Phosphatgruppen sowie funktonalisierte Perluorkohlenwasserstoffe mit anderen Gruppen oder Atomen, über die eine Verbindung zu Nukleosiden, Nukleotiden, Oligonukleotiden, Nukleinsäuren, modifizierte Nukleosiden, modifizierten Nukleotiden, modifizierten Oligonukleotiden, modifizierten Nukleinsäuren, Peptid-Nukleosiden, Peptid-Nukleotiden, Peptid-Oligonukleotiden, Peptid-Nukleinsäuren oder pharmazeutische Substanzen hergestellt werden kann.

Bevorzugte Linkermoleküle basieren auf Hydroxyl- oder Aminogruppen, Carboxylgruppen, Ester, Ether, Thioether, Thioester, Carbonsäureamide, Verbindungen mit Mehrfachbindungen, Carbamate, Disulfidbrücken und Hydrazide, Halogenalkane, Sulhydryl-, Aldehyd- Keto-, Carboxyl-, Ester- und Säureamidgruppen, Thiolen, Aminen, Iminen, Hydrazinen, oder Disulfidgruppen, Glycerol, Succinylglycerol, Orthoester, Phosphorsäurediester und Vinylether, wobei Ester- und Ethergruppen und Disulfidbrücken ganz besonders bevorzugt sind.

In einer bevorzugten Ausführungsform ist die inertisierte Materialoberfläche dadurch gekennzeichnet, dass die kovalente Bindung durch ein Linker- oder Spacermolekül erfolgt, wobei das Linker- oder Spacermolekül bevorzugt auf Hydroxyl- oder Aminogruppen, Carboxylgruppen, Ester, Ether, Thioether, Thioester, Carbonsäureamide, Verbindungen mit Mehrfachbindungen, Carbamate, Disulfidbrücken und Hydrazide, Halogenalkane, Sulhydryl-, Aldehyd- Keto-, Carboxyl-, Ester- und Säureamidgruppen, Thiolen, Aminen, Iminen, Hydrazinen, oder Disulfidgruppen, Glycerol, Succinylglycerol, Orthoester, Phosphorsäurediester und Vinylether basiert, wobei Ester- und Ethergruppen und Disulfidbrücken ganz besonders bevorzugt sind.

Das Linkermolekül kann vorteilhafterweise zur Anbindung von weiteren Funktionsmolekülen genutzt werden, die beispielsweise eine elektrische Leitfähigkeit erzeugen oder die den Bewuchs mit Zellen forcieren. Andere Beispiele sind die Anbindung von Wachstumsfaktoren oder die Anbindung von Molekülen, die Funktionen tragen, die sich von den Umgebungseigenschaften abheben.

Beispiele für derart geeignete wären Moleküle im Bereich der molekularen Elektronik oder Sensorik (Ferroceenverbindungen und andere), Wachstumsfaktoren wie Fibroblast Growth Factor (FGF), Vascular Endothelial Growth Factor (VEGF), Nervenwachstumsfaktor (Nerve Growth Factor, NGF), metallische leitfähige Verbindungen oder Moleküle biokompatibler Grenzflächen zwischen anorganischen und organischen Materialien.

In einer weiteren bevorzugten Ausführungsform betrifft die Erfindung die Verwendung einer mit den beschriebenen Verfahren inertisierten Materialoberfläche, bevorzugt einer Keramik-, Metall- oder Kunststoffoberfläche in einem Medizinprodukt, bevorzugt in einem Implantat in dem bevorzugt eine Zellanlagerung ganz oder partiell unerwünscht ist, besonders bevorzugt in einem Herzimplantat, einem Stent, einer Prothese, einem Gelenk, in Zahnimplantaten oder in ein Medizintechnikapparatur durch welche Substanzen, insbesondere Blut oder Blutbestandteile geleitet werden.

Vorteilhafterweise zeichnen sich Medizinprodukte, in welchen die inertisierten Materialoberflächen, bevorzugt die Keramik-, Metall- oder Kunststoffoberflächen verwandt werden, durch eine besonders hohe Biokompatibilität aus und weisen eine äußert geringes Risiko für Komplikationen auf.

Ein Implantat bezeichnet im Sinne der Erfindung bevorzugt eine medizinische Vorrichtung um eine biologische Struktur zu ersetzen, unterstützen oder zu verbessern. Ein Zahnimplant bezeichnet bevorzugt ein Implantat für den Mund- oder Kieferraum und kann bevorzugt sowohl Zahnstrukturen ersetzen, als auch diese unterstützen. Unter einem Herzimplantat wird bevorzugt eine Vorrichtung verstanden, welche die Funktion des menschlichen Herzens nachahmt, beispielsweise zur Überbrückung der Zeit, die ein Patient auf ein Spenderherz warten muss.

Aufgrund der inerten Eigenschaften der Keramik- oder Metalloberflächen zeichnen sich die Medizinprodukte durch besonders geringe Ablagerungen aus und können somit über lange Zeiträume im Körper getragen werden, ohne dass Ablagerungen oder Verschleiß zu Komplikationen führen.

In einer weiteren bevorzugten Ausführungsform betrifft die Erfindung die Verwendung der inertisierten Materialoberflächen, bevorzugt der Keramik- oder Metalloberflächen, in einem Produkt der Elektrotechnik, insbesondere der Mikroelektronik. In der Elektrotechnik und Mikroelektronik werden vielfältig Hochleistungskeramiken und Metalle als Materialien für immer komplexer werdende Bauteile eingesetzt. Aufgrund statischer Aufladungen, kommt es hierbei häufig zu Material- oder Staubanlagerungen, welche selbst als Mikroanlagerungen die Leistung und Lebensdauer der Geräte vermindern können. Mit den erfindungsgemäßen inertisierten Keramik- oder Metalloberflächen kann dies wirksam vermieden werden. Vorteilhafterweise stellen die beschriebenen Verfahren, insbesondere unter Verwendung kurzkettiger perfluorierter Verbindungen, zudem sicher, dass sich die gewünschten elektrischen Eigenschaften der Keramik- oder Metalloberflächen durch die Inertisierung nicht verändern. Es ist bekannt, dass oberhalb von perfluorierten Grenzflächen das sonst besehende elektrische Feld eines Materials vergleichsweise sehr klein ist. Eine elektrostatische Anziehung kann sich dadurch nicht entwickeln.

In einer weiteren bevorzugten Ausführungsform betrifft die Erfindung die Verwendung der inertisierten Materialoberflächen in Fenstergläsern, Einrichtungsgegenständen, sanitären Anlagen oder Gebäuden. Vorteilhafterweise lässt sich das beschriebene Verfahren auch zur Inertisierung von Glasoberflächen oder Keramiken einsetzen, welche typischerweise in Fenstergläsern, Einrichtungsgegenständen, sanitären Anlagen oder Gebäuden verwandt werden. Derart inertisierte Materialoberflächen zeichnen sich in den vorgenannten Anwendungen dadurch aus, dass sich kein oder nur kaum Schmutz an diesen anlagern kann. Hierdurch kann der Reinigungsaufwand deutlich verringert werden.

Im Folgenden soll die Erfindung an Hand von Beispielen näher erläutert werden, ohne auf diese beschränkt zu sein.

Es wird darauf hingewiesen, dass verschiedene Alternativen zu den beschriebenen Ausführungsformen der Erfindung verwendet werden können, um die Erfindung auszuführen und zu der erfindungsgemäßen Lösung zu gelangen. Die erfindungsgemäßen Verfahren und inertisierte Keramik- und Metalloberflächen beschränken sich in ihren Ausführungen somit nicht auf die genannten bevorzugten Ausführungsformen. Vielmehr ist eine Vielzahl von Ausgestaltungsvarianten denkbar, welche von der dargestellten Lösung abweichen können. Ziel der Ansprüche ist es, den Schutzumfang der Erfindung zu definieren. Der Schutzumfang der Ansprüche ist darauf gerichtet, die erfindungsgemäßen Verfahren und inertisierte Materialoberflächen, bevorzugt die Keramik-, Metall- und Kunststoffoberflächen sowie äquivalente Ausführungsformen von diesen abzudecken. Es werden die Gewichtsteile der Komponenten mit teilweise mit der Abkürzung ,Teile' angegeben.

### Beispiele für Inertisierungswege von Keramik- und Metalloberflächen mit funktionalisierten perfluorierten Verbindungen:

Die Modifizierungen der Keramik- und Metalloberflächen mit funktionalisierten perfluorierten Verbindungen sind auf verschiedene Weise umsetzbar:
1) Voraktivierung der Oberflächen durch Säuren (z.B. mit Schwefelsäure oder Flusssäure), durch andere Agenzien, durch Laserbehandlung, durch Korona- , Ozon- oder Plasmabehandlung oder andere Verfahren und anschließende Reaktion mit funktionalisierten perfluorierten Verbindungen
2) direkte Reaktion mit funktionalisierten perfluorierten Verbindungen
3) Beschichtung mit einer Schicht, an der funktionalisierte perfluorierte Verbindungen reagieren können

Durch eine selektive Oberflächenbehandlung (z.B. Laserbehandlung) oder durch ein selektiv erzeugtes Druckbild und die damit verbundene Zugänglichkeit oder Nicht-Zugänglichkeit für die Reaktionen mit den funktionalisierten perfluorierten Verbindungen kann ein definiertes Muster zwischen Bereichen unterschiedlicher Eigenschaften (z.B. hohe und sehr niedrige Oberflächenenergie) erzeugt werden.

### Zu 1) und 2) (Vor-)Aktivierunq der Oberflächen mit Schwefelsäure oder Flusssäure und anschließende Reaktion mit einer funktionalisierten perfluorierten Verbindung oder direkte Reaktion:

Ausgehend von der abgebildeten Elementarzelle der kristallinen Struktur des Zirkonium(IV)-oxids sind verschiedene Modifizierungsverfahren möglich, wobei mehrere Reaktionen zur Veränderung der Oberflächenstruktur zur Option stehen.

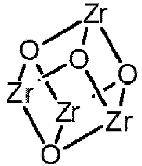

Da Hochleistungskeramiken aus Zirkonium(IV)-oxid und andere eine hohe chemische und thermische Stabilität besitzen, kann es bevorzugt sein, eine Oberflächenaktivierung mittels Fluorwasserstoffsäure (Flusssäure) und/oder konzentrierter Schwefelsäure durchzuführen. In einigen Fällen kann aber auch darauf verzichtet werden (direkte Reaktion).

Durch die Aktivierung werden zunächst die zur Oberfläche ausgerichteten Sauerstoffatome protoniert. Findet eine weitere Protonierung statt, können die Sauerstoffatome als Wasser abgespalten werden (siehe Reaktionsgleichung 1).

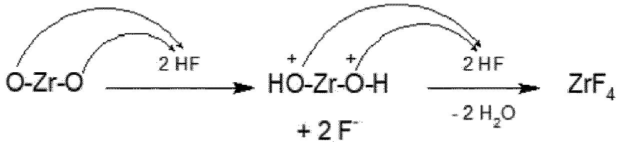

### Reaktionsgleichung 1: Protonierung der Sauerstoffatome des ZrO₂

Durch das Abspalten des Sauerstoffs kann das Zirkoniumatom als vierwertiges Ion (Zr⁴⁺) vorliegen und mit den Fluoridionen in eine ionische Verbindung treten (Salz). Dadurch löst sich das Zirkoniumion aus der Oberflächenstruktur der Keramik heraus und wird "ausgespült".

So können in einer Vorbehandlung verschiedene aktivierte Zustände der ZrO₂-Oberflächenstruktur erzeugt werden. Diese sind Ausgangspunkt für die Modifizierung mit funktionalisierten perfluorierten Verbindungen.

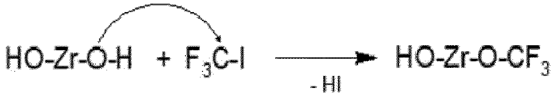

### Reaktionsgleichung 2: nukleophile Substitution an der aktivierten Keramikoberfläche

Wie Reaktionsgleichung 2 zeigt, kann der aktivierte erste protonierte Zustand der Keramik nukleophil am positivierten perfluorierten Methyliodid-Kohlenstoff angreifen, sodass es zu einer Anlagerung einer perfluorierten Methylgruppe kommt. Der gebildete perfluorierte "Ether" ist für Körperzellen schwerer zu spalten als eine nicht-perfluorierte Methylgruppe, da die enthaltenen Fluoratome durch ihre geringe relative Atommasse, hohe Elektronegativität und ihren geringen Radius (lonenradius) schlechte Abgangsgruppen darstellen und somit nicht abgespalten werden.

Außerdem können Methylgruppen mit ihren Wasserstoffatomen sowohl van-der-Waals-Wechselwirkungen als auch Wasserstoffbrückenbindungen ausbilden, was bei den perfluorierten Resten deutlich erschwert ist.

Durch diesen Umstand ist es möglich, auch andere perfluorierte Gruppen zur Modifizierung einzusetzen, die mittels grundlegender Reaktionsmechanismen an die aktivierte Keramikoberfläche binden.

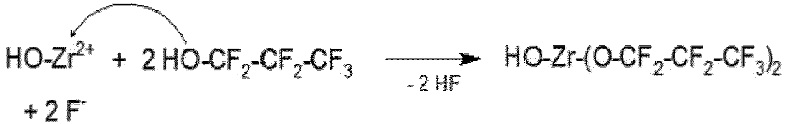

### Reaktionsgleichung 3: aktivierte Keramikoberfläche als Elektrophil

Wie Reaktionsgleichung 3 zeigt, ist auch die ionische aktivierte Zwischenstufe als elektrophiler Reaktand einsetzbar. Hierbei wird das Zirkoniumion nukleophil vom perfluorierten Alkohol angegriffen. Unter Abspaltung von Fluorwasserstoffsäure (die für eine weitere Oberflächenaktivierung in den Reaktionsprozess wiedereintreten kann) bildet sich der perfluorierte "Diether".

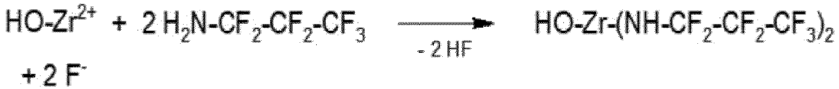

### Reaktionsgleichung 4: aktivierte Keramikoberfläche als Elektrophil

Analog zur Reaktionsgleichung 3 zeigt auch Reaktionsgleichung 4 eine Reaktionsmöglichkeit, bei der die aktivierte Keramikoberfläche als elektrophiler Reaktand Verwendung findet. Als nukleophiler Reaktionspartner ist ein primäres Amin eingesetzt. Auch perfluorierte sekundäre Amine können diesen Reaktionstyp erfüllen. Das Amin greift mittels freiem Elektronenpaar des Stickstoffs das positiv geladene Zirkonium an und bindet an dieses. Es spaltet sich ein Proton (H⁺) ab und bildet mit dem Fluoridion HF, was wieder zur Oberflächenaktivierung zur Verfügung steht.

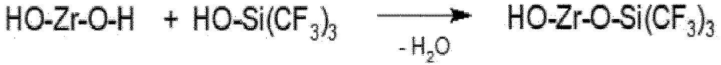

### Reaktionsgleichung 5: Anbindung eines perfluorierten Silans an die aktivierte Keramikoberfläche

In Anlehnung an "Silikone" können perfluorierte Silane genutzt werden. Dieser Reaktionstyp entspricht einer Kondensation, bei der Wasser als Nebenprodukt abgespalten wird (siehe Reaktionsgleichung 5 & 6). Der Silansauerstoff greift nukleophil das positiv polarisierte Zirkonium an. Es wird ein H⁺ und eine OH-Gruppe als Wasser abgespalten.

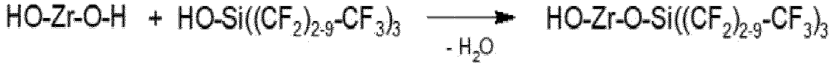

### Reaktionsgleichung 6: Anbindung eines perfluorierten Silans an die aktivierte Keramikoberfläche

Wie in Reaktionsgleichung 5 greift der Silansauerstoff nukleophil das Zirkonium an und es wird ein H⁺ und eine OH-Gruppe als OH⁻ abgespalten, die das Nebenprodukt Wasser bilden.

Das Einbringen von Silanen oder anderen Halbmetallen oder Metallen (Dotieren) wird zu einer Erhöhung der elektrischen Leitfähigkeit genutzt. Zirkonium(IV)-oxid kann bei hohen Temperaturen Sauerstoffionen elektrolytisch transportieren. Dadurch entsteht eine elektrische Spannung. Durch die Dotierung wird eine Verbesserung der Leitfähigkeit bei niedrigeren Temperaturen erreicht.

Im Gegensatz dazu wird mit dem Anbringen perfluorierter Organyle auf der Zirkonium(IV)-oxid-Oberfläche eine Leitung des elektrischen Stroms verringert oder verhindert. Diese Isolatorwirkung ermöglicht in der Elektrotechnik (Mikroelektronik) eine präzise Herstellung von Leitsystemen für die Leitung des elektrischen Stroms.

HO-Zr²⁺ + 2H₂ + 2CF₂=CF₂ → HO-Zr(CF₂H-CF₂H)₂

### Reaktionsgleichung 7: Addition und Hydrierung von Tetrafluorethen an der aktivierten Keramikoberfläche

Mittels der Doppelbindung des Tetrafluorethens findet eine Addition am elektrophilen Zirkonium statt. Durch anschließende Hydrierung (Anlagerung von H₂) wird die vormalige Ethengruppe reduziert.

Reaktionsgleichung 7 zeigt ein Analogon zum ersten Schritt des Ziegler-Direkt-Verfahrens. Dabei wird additiv das Tetrafluorethen an die aktivierte Zirkoniumoxid-Oberfläche angelagert und hydriert.

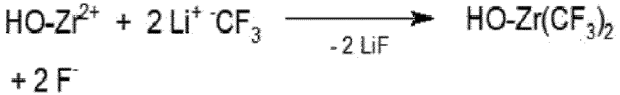

### Reaktionsgleichung 8: Reaktion eines perfluorierten Metallorganyls mit der aktivierten Keramikoberfläche

Die Reaktionsgleichung 8 zeigt die Reaktion eines perfluorierten Metallorganyls (Lithium-Organyl, ein Kupfer-Lithium-Organyl wäre beispielsweise ebenfalls möglich). Das perfluorierte Organyl ist ein starkes Nukleophil, das das positive Zirkoniumion an der aktivierten Keramikoberfläche nukleophil angreift. Zusätzlich begünstigt wird diese Reaktion durch die Bildung des Salzes LiF (Lithiumfluorid), das eine stark negative Gitterenergie besitzt.

HO-Zr-OH + 2LiCF₃ → LiO-Zr(CF₃)₂-OLi

### Reaktionsgleichung 9: Reaktion des aktivierten nicht-ionischen Zustands der Keramikoberfläche mit einem perfluorierten Metallorganyl (Lithiumorganyl)

Die bereits in Reaktionsgleichung 8 beschriebene Reaktion kann auch mit dem nicht-ionischen Zwischenzustand der aktivierten Zirkoniumoxid-Oberfläche durchgeführt werden. Dabei wird eine weitere Aktivierung durch die Metallierung des Oberflächen-Sauerstoffs erreicht, wobei sich bereits zwei perfluorierte Organylreste an das Zirkonium-Zentrum anlagern. Diese lithiierte Oberfläche ist in der Lage, weitere nukleophile Reaktionen einzugehen.

Auch für weitere Keramiken und Hochleistungskeramiken aus Metalloxiden gelten die oben ausgeführten Reaktionen in gleicher Weise.

So eignet sich ebenfalls das in einer Korund-Struktur verwendete Aluminiumoxid für diese Reaktionen.

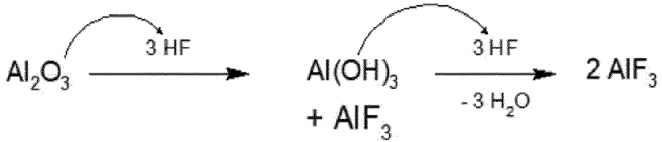

### Reaktionsgleichung 10: Reaktion der Aluminiumoxid-Keramik mit Fluorwasserstoffsäure

Der Sauerstoff der Oberflächenstruktur der Keramik wird durch die Flusssäure protoniert, sodass als Zwischenstufe Al(OH)₃ entsteht sowie AlF₃. Durch weitere Protonierung der OH-Gruppen spaltet sich Wasser ab und es entsteht das reaktivere AlF₃.

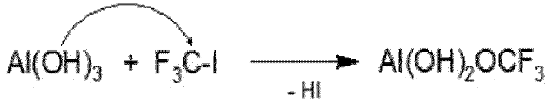

### Reaktionsgleichung 11: Reaktion der aktivierten Keramikoberfläche mit einem perfluorierten Halogenalkyl

Der Sauerstoff des Al(OH)₃ greift nukleophil das positiv polarisierte C-Atom des Trifluoriodmethans an. Iod wird als lodid abgestalten und bildet mit dem Proton der OH-Gruppe das Nebenprodukt lodwasserstoff (HI).

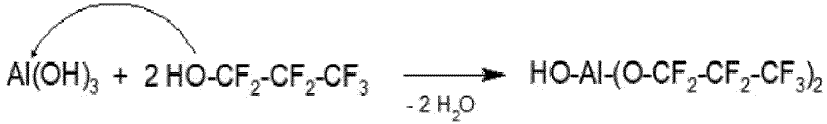

### Reaktionsgleichung 12: Reaktion der aktivierten Keramikoberfläche mit einem perfluorierten Alkohol

Der Alkoholsauerstoff des perfluoriertien Alkohols greift nukleophil das Aluminium an. Es wird eine OH-Gruppe als OH⁻ sowie das Alkoholproton (H⁺) abgespalten, die Wasser als Nebenprodukt bilden.

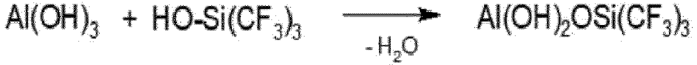

### Reaktionsgleichung 13: Anbindung eines perfluorierten Silans an die aktivierte Keramikoberfläche

Der Silansauerstoff greift nukleophil das Aluminium an und es wird OH⁻ sowie das Proton der Silan-OH-Gruppe abgespalten, sodass sich Wasser als Nebenprodukt bildet.

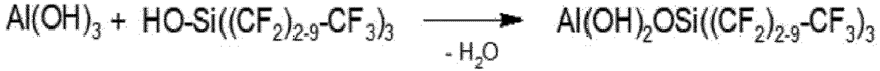

### Reaktionsgleichung 14: Anbindung eines perfluorierten Silans an die aktivierte Keramikoberfläche

In Reaktionsgleichung 14 verläuft der Mechanismus wie in Gleichung 13. Der Silansauerstoff greift nukleophil das Aluminium an und es wird OH⁻ sowie das Proton der Silan-OH-Gruppe abgespalten, sodass sich Wasser als Nebenprodukt bildet.

Al(OH)₃ + 3LiCF₃ → Al(CF₃)₃

### Reaktionsgleichung 15: Reaktion der aktivierten Keramikoberfläche mit einem perfluorierten Lithiumorganyl

Das in Reaktionsgleichung 15 gezeigte Lithiumorganyl ist stark polarisiert. Das negativ polarisierte Organyl-C-Atom greift nukleophil das Aluminium an. Es wird OH⁻ abgespalten, welches mit Li⁺ LiOH als Nebenprodukt bildet.

Titan(IV)-oxid (TiO₂) ist eine weitere Hochleistungskeramik, die durch die oben genannten Reaktionen modifiziert werden kann.

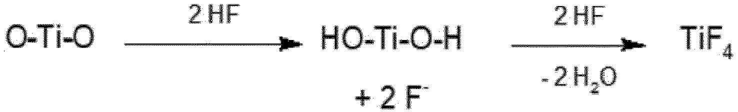

### Reaktionsgleichung 16: Protonierung der Sauerstoffatome des TiO₂

Die Sauerstoffatome der TiO₂-Oberfläche werden durch HF protoniert bis das reaktivere TiF₄ entsteht.

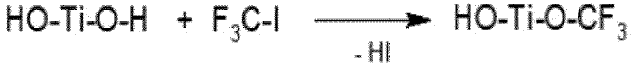

### Reaktionsgleichung 17: nukleophile Substitution an der aktivierten Keramikoberfläche

Das in Reaktionsgleichung 17 gezeigte Trifluoriodmethan ist polarisiert, sodass ein Sauerstoff der aktivierten Keramikoberfläche nuklephil das positiv polarisierte C-Atom des Trifluoriodmethans angreift und lodid abgespalten wird. Dieses lagert sich mit dem abgespaltenen Proton zu lodwasserstoff (HI) als Nebenprodukt zusammen.

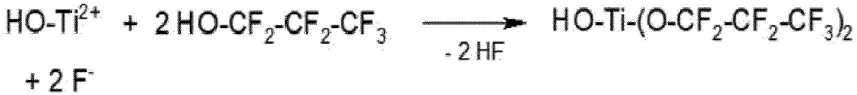

### Reaktionsgleichung 18: aktivierte Keramikoberfläche als Elektrophil

Die in Reaktionsgleichung 18 gezeigte aktivierte Keramikoberfläche reagiert als Elektrophil und wird als solches nukleophil vom Sauerstoff des perfluorierten Alkohols angegriffen. Es wird das Proton (H⁺) der Alkoholgruppe abgespalten, welches sich mit einem Fluoridion zu HF als Nebenprodukt zusammenlagert, welches für weitere Oberflächenaktivierung zur Verfügung steht.

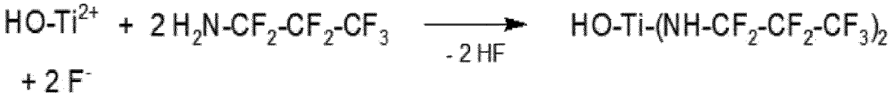

### Reaktionsgleichung 19: aktivierte Keramikoberfläche als Elektrophil

In Reaktionsgleichung 19 fungiert das perfluorierte primäre Amin als Nukleophil. Der Aminstickstoff greift nukleophil das positve Titan an. Es wird ein H⁺ der Aminogruppe abgespalten, das mit einem F⁻ das Nebenprodukt HF bildet.

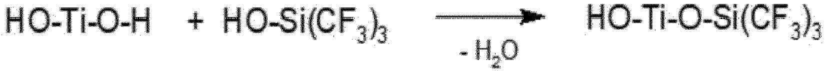

### Reaktionsgleichung 20: Anbindung eines perfluorierten Silans an die aktivierte Keramikoberfläche

In Reaktionsgleichung 20 greift der Silansauerstoff das Titan der Keramikoberfläche nukleophil an. Es wird OH⁻ abgespalten, das mit H⁺ Wasser als Nebenprodukt bildet.

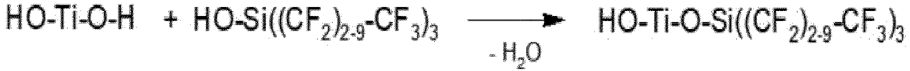

### Reaktionsgleichung 21: Anbindung eines perfluorierten Silans an die aktivierte Keramikoberfläche

In Reaktionsgleichung 21 greift der Silansauerstoff das Titan der Keramikoberfläche nukleophil an. Es wird OH⁻ abgespalten, das mit H⁺ Wasser als Nebenprodukt bildet.

HO-Ti²⁺ + 2H₂ + 2CF₂=CF₂ → HO-Ti(CF₂H-CF₂H)₂

### Reaktionsgleichung 22: Addition und Hydrierung von Tetrafluorethen an der aktivierten Keramikoberfläche

Mittels der Doppelbindung des Tetrafluorethens findet eine Addition am elektrophilen Titan statt. Durch anschließende Hydrierung (Anlagerung von H₂) wird die vormalige Ethengruppe reduziert zu einer Tetrafluorethangruppe.

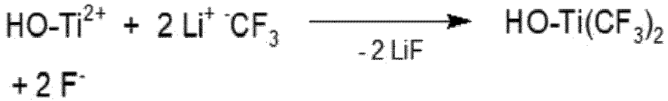

### Reaktionsgleichung 23: Reaktion eines perfluorierten Metallorganyls mit der aktivierten Keramikoberfläche

Das in Reaktionsgleichung 23 gezeigte Lithiumorganyl ist stark polarisiert. Das negativ polarisierte Organyl-C-Atom greift nukleophil das Titan an. Es wird OH⁻ abgespalten, welches mit Li⁺ LiOH als Nebenprodukt bildet.

HO-Ti-OH + 2LiCF₃ → LiO-Ti(CF₃)₂-OLi

### Reaktionsgleichung 24: Reaktion des aktivierten nicht-ionischen Zustands der Keramikoberfläche mit einem perfluorierten Metallorganyl (Lithiumorganyl)

Reaktionsgleichung 24 zeigt wie Glechung 23 eine nukleophile Anlagerung des negativ polarisierten Organyls an das Titan. Zusätzlich bindet Li⁺ an die Sauerstoffatome der Keramikoberfläche, sodass eine weiterhin reaktive spezies entsteht.

### Zu 3) Beschichtung mit einer Schicht, an der funktionalisierte perfluorierte Verbindungen reagieren können

Statt die Keramik-, oder Metalloberfläche für eine Reaktion mit den funktionalisierten perfluorierten Verbindungen zu aktivieren, kann alternativ eine Beschichtung mit zugänglichen funktionellen Gruppen aufgebracht werden (vgl. Beispiel 1 und 3 der beispielhaften Inertisierungswege von Kunststoffoberflächen mittels funktionalisierter perfluorierter Verbindungen)

So kann es bevorzugt sein, dass die Keramik mit einer Polyurethanverbindung beschichtet wird, welche OH-Gruppen enthält und sich gut mit der Oberfläche der Keramik verbindet. Anschließend kann über die OH-Gruppen eine Reaktion mit funktionalisierten perfluorierten Verbindungen herbeigeführt, wobei in diesem Fall eine perfluorierte Verbindung mit Isocyanatgruppe genutzt wird.

In einem weiteren Beispiel wird die Keramik mit einer Polymerverbindung beschichtet, die NH2-Gruppen enthält und sich gut mit der Oberfläche der Keramik verbindet. Anschließend wird über die NH2-Gruppen eine Reaktion mit funktionalisierten perfluorierten Verbindungen herbeigeführt, wobei in diesem Fall eine perfluorierte Verbindung mit Aldehydgruppe genutzt wird.

In einem weiteren Beispiel wird die Keramik mit einer Polymerverbindung beschichtet, die eine Azid-Alkin-Cycloaddition ermöglicht. Diese Polymerverbindung enthält Azidgruppen, über die die funktionalisierten perfluorierten Verbindungen mittels Alkin-Gruppe gebunden werden. Andere Varianten der Klick-Chemie sind ebenfalls nutzbar.

### Beispielhafte Inertisierungswege von Kunststoffoberflächen mittels funktionalisierter perfluorierter Verbindungen:

Die Inertisierungen von Kunststoffoberflächen sind auf verschiedene Weise umsetzbar:
1) Direkte chemische Anbindung der funktionalisierten perfluorierten Verbindungen über funktionelle Gruppen des Kunststoffs
2) Aktivierung der Kunststoffoberfläche (durch Plasma-, Korona- oder Laserbehandlung, durch Röntgenstrahlung oder UV-Bestrahlung, durch HF-Behandlung oder chemische Substanzen oder andere Vorgänge, durch die die Kunststoffoberfläche aktiviert werden kann) und anschließende chemische Anbindung der funktionalisierten perfluorierten Verbindungen
3) Beschichtung des Kunststoffs mit einer Schicht, die der chemischen Anbindung der funktionalisierten perfluorierten Verbindungen zugänglich ist mit anschließender Anbindung dieser Moleküle an diese Schicht

Durch eine selektive Oberflächenbehandlung (z.B. Laserbehandlung, andere) oder durch ein selektiv erzeugtes Druckbild und die damit verbundene Zugänglichkeit oder Nicht-Zugänglichkeit für die Reaktionen mit den funktionalisierten perfluorierten Verbindungen kann ein definiertes Muster zwischen Bereichen unterschiedlicher Eigenschaften (z.B. hohe und niedrige Oberflächenenergie oder andere räumlich differenzierte Eigenschaften) erzeugt werden.

### 1) Beispiele für eine direkte chemische Anbindung der funktionalisierten perfluorierten Verbindungen an funktionelle Gruppen von Kunststoffoberflächen sowie 3) Beispiele für Anbindung an Schichten, die der chemischen Anbindung der funktionalisierten perfluorierten Verbindungen zugänglich sind:

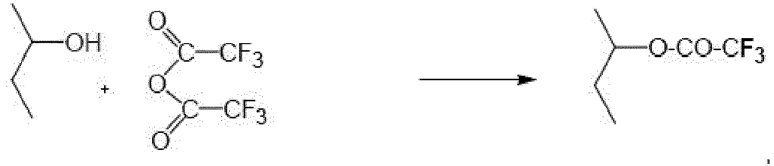

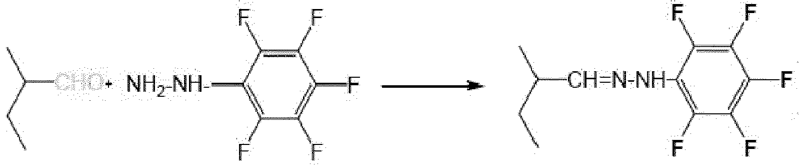

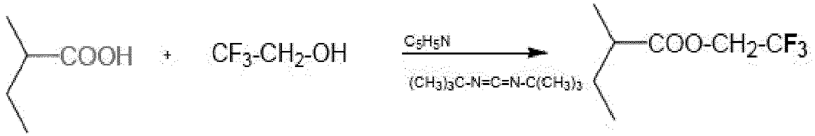

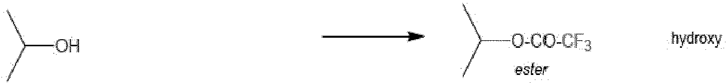

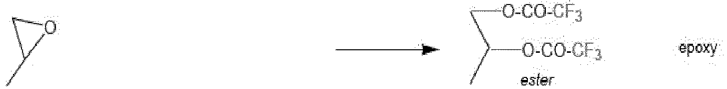

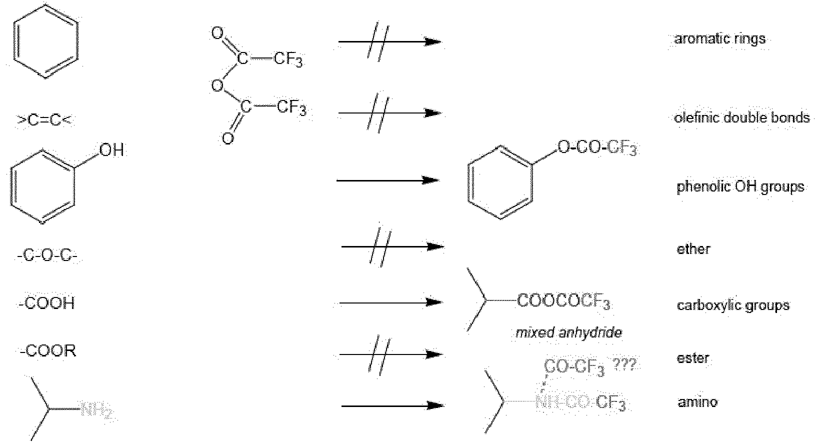

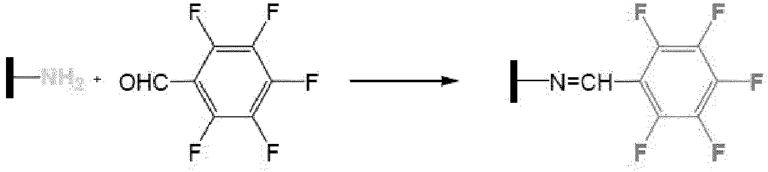

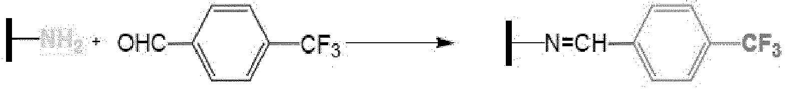

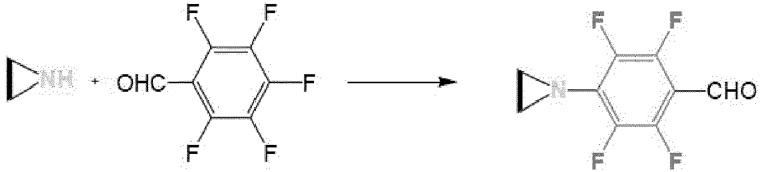

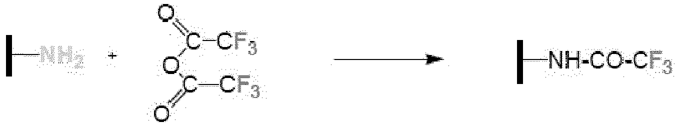

Beispiele für prinzipielle Anbindungsmöglichkeiten von funktionalisierten perfluorierten Verbindungen an funktionelle Gruppen einer Kunststoffoberfläche oder einer Beschichtung sind unter anderem:

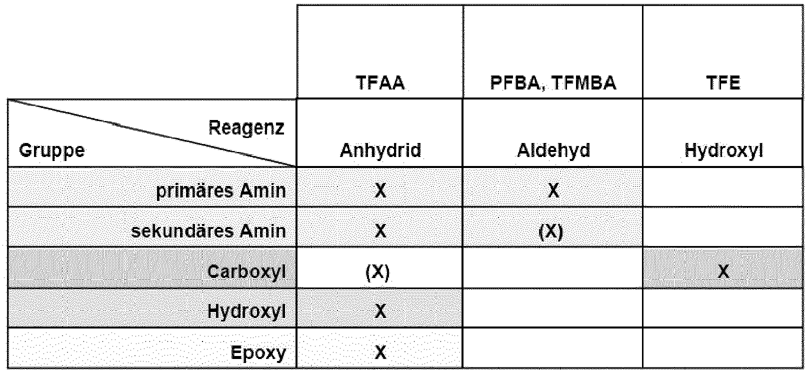

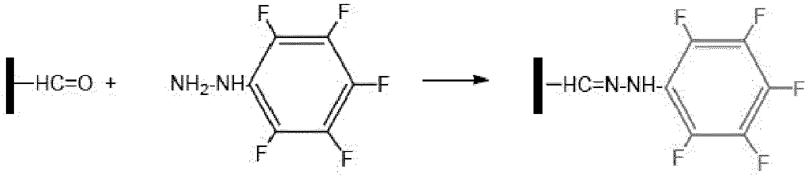

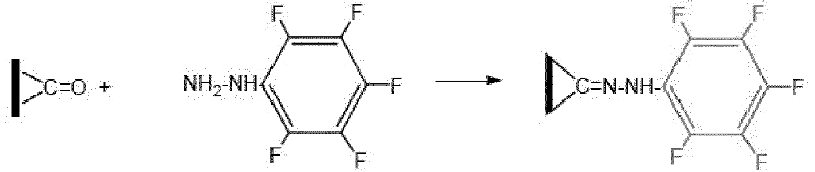

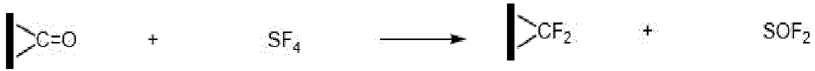

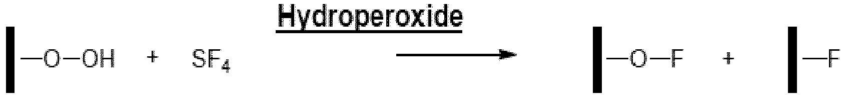

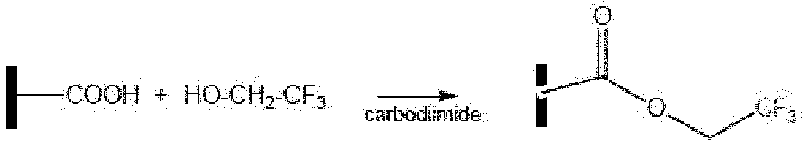

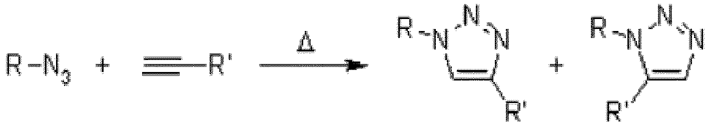

R oder R' = funktionalisierte perfluorierte Verbindung

Eine weitere Möglichkeit ist die Quervernetzung von funktionalisierten perfluorierten Verbindungen mit Doppelbindungen (z.B. perfluorierte Sulfonsäuren oder perfluorierte Carbonsäuren oder perfluorierte Monomere von Kunststoffen wie z.B. Acrylate) mit Kunststoffen, die ebenfalls Doppelbindungen enthalten (z.B. Polyester) unter UVA-Bestrahlung. 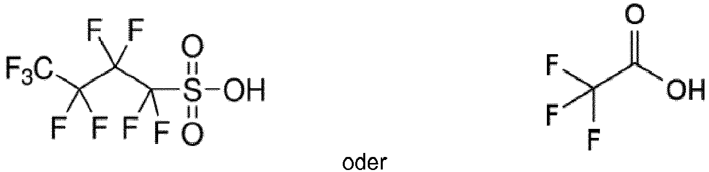 oder 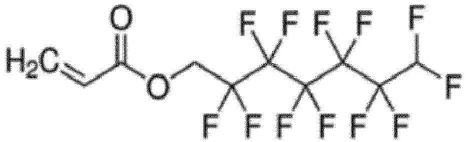 oder 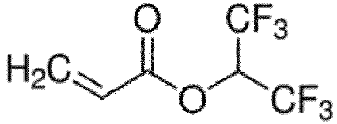 und 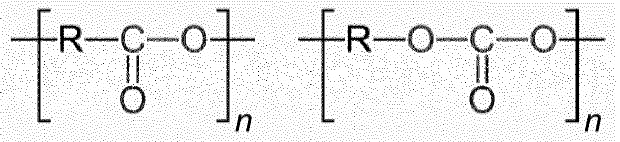 oder 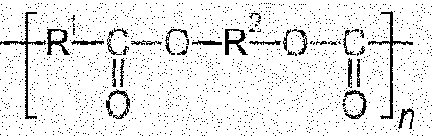 oder 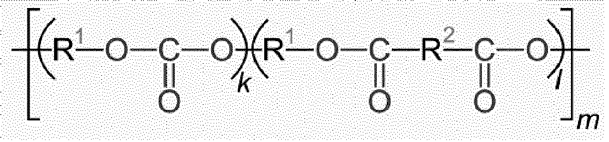 oder andere Kunststoffe mit Doppelbindung

### 2) Beispiele für die Aktivierung der Kunststoffoberfläche (durch Plasma-, Korona- oder Laserbehandlung, durch Röntgenstrahlung oder UV-Bestrahlung, durch chemische Substanzen oder andere Vorgänge, durch die die Kunststoffoberfläche aktiviert werden kann) und anschließende chemische Anbindung der funktionalisierten perfluorierten Verbindungen:

Bei einer energiereiche O2-Plasma-UV-Strahlung werden beispielsweise Wasserstoffatome aus der Kunststoffoberfläche ausgeschlagen. Diese werden entweder durch Sauerstoffatome ersetzt, die in der Regel als Radikal vorliegen, oder es bilden sich lone. Diese aktivierten Kunststoffoberflächen können anschließend sehr gut mit funktionalisierten perfluorierten Verbindungen reagieren.

Prinzipiell eignen sich bevorzugt perfluorierte Verbindungen, an deren funktionelle Gruppe ein nukleophiler Angriff unternommen werden kann oder auch positiv geladene perfluorierte Ionen. Aber auch andere chemische Verbindungen sind möglich.

Beispiele für bevorzugte funktionalisierte perfluorierte Verbindungen:

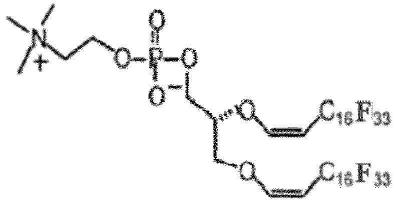

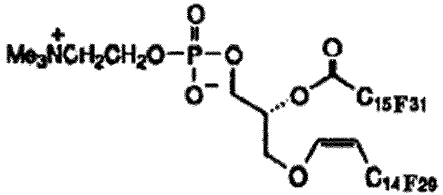

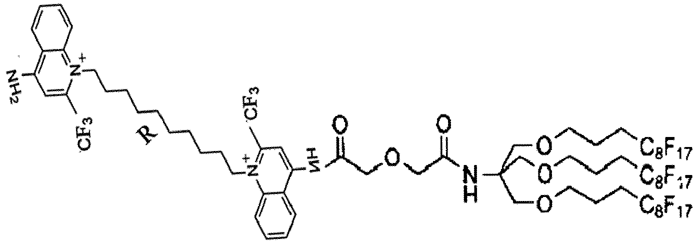

Ohne einen perfluorierten Molekülteil wären die dargestellten Moleküle biologisch abbaubar. Nach Anbindung an den Kunststoff schirmen die abstehenden perfluorierten Ketten die Anbindung von abbauenden Enzymen ab.

### 3) Beispiele für Beschichtung des Kunststoffs mit einer Schicht, die der chemischen Anbindung der funktionalisierten perfluorierten Verbindungen zugänglich ist mit anschließender Anbindung dieser Moleküle an diese Schicht:

Beispielsweise wird der Kunststoff mit einer Polyurethanverbindung beschichtet, die OH-Gruppen enthält. Anschließend wird über die OH-Gruppen eine Reaktion mit funktionalisierten perfluorierten Verbindungen herbeigeführt wie oben unter 1) / 3) beispielhaft dargestellt.

In einem weiteren Beispiel wird der Kunststoff mit einer Polyacrylamidverbindung beschichtet, die NH2-Gruppen enthält. Anschließend wird über die NH2-Gruppen eine Reaktion mit funktionalisierten perfluorierten Verbindungen herbeigeführt, wie oben unter 1) / 3) beispielhaft dargestellt.

In einem weiteren Beispiel wird der Kunststoff mit einer Schicht beschichtet, die eine Azid-Alkin-Cycloaddition ermöglicht. Diese Polymerverbindung enthält Azidgruppen, über die die funktionalisierten perfluorierten Verbindungen mittels Alkin-Gruppe gebunden werden. Andere Varianten der Klick-Chemie sind ebenfalls nutzbar.

## Patentansprüche

1. Verfahren zur Inertisierung von Materialoberflächen umfassend folgende Schritte
a) Bereitstellung einer Materialoberfläche
b) Bereitstellung einer funktionalisierten perfluorierten Verbindung
c) Reaktion der funktionalisierten perfluorierten Verbindung mit der Oberfläche zur Ausbildung einer kovalenten Bindung, einer Ionenbeziehung oder einer Metallbindung zwischen der perfluorierten Verbindung und der Materialoberfläche.

2. Verfahren gemäß dem vorherigen Anspruch
**dadurch gekennzeichnet, dass**
die Materialoberfläche eine Metall-, Keramik- oder Kunststoffoberfläche ist.

3. Verfahren gemäß dem vorherigen Anspruch
**dadurch gekennzeichnet, dass**
die funktionalisierte perfluorierte Verbindung eine perfluorierte Verbindung und eine funktionelle Gruppe umfasst, wobei die perfluorierte Verbindung höchstens 20 vollständig fluorierte Atome umfasst.

4. Verfahren gemäß einem der vorherigen Ansprüche
**dadurch kennzeichnet, dass**
vor dem Schritt c) eine Aktivierung der Oberfläche erfolgt, wobei die Aktivierung bevorzugt durch Säure-, Laser-, Korona-, Ozon- oder Plasmabehandlung erfolgt.

5. Verfahren einem der vorherigen Ansprüche
**dadurch kennzeichnet, dass**
für Teilbereiche der Materialoberfläche eine unterschiedliche Aktivierung erfolgt.

6. Verfahren einem der vorherigen Anspruch
**dadurch gekennzeichnet, dass**
die funktionalisierten perfluorierten Verbindungen eine perfluorierte Verbindung mit mindestens einer funktionellen Gruppe ist, bevorzugt einer nukleophilen Abgangsgruppe, besonders bevorzugt ausgewählt aus einer Gruppe umfassend Br, I, Cl, H, N, O, S, P, Hydroxyl-, Amino-, Carboxylgruppen,Carbonsäuren, Thiocarbonsäuren, Sulfonsäuren, Sulfinsäuren, Carbonsäurehalogeniden, Sulfonsäurehalogeniden, Säureamide, Carbonsäureamiden, Sulfonsäureamiden, Alkohole, Phenolen, Hydrazinen, Thiolen, Aminen, Iminen, Hydrazinen, Isocyanaten, Thiocyanaten, Isothiocyanaten.

7. Verfahren einem der vorherigen Anspruch
**dadurch gekennzeichnet, dass**
die funktionalisierten perfluorierten Verbindungen eine perfluorierte Verbindung mit mindestens einer funktionellen Gruppe ist, die mindestens eine Doppelbindung umfasst und durch UV-Quervernetzung angebunden werden kann, eine perfluorierte Verbindung mit mindestens einer funktionellen Gruppe ist, welche an Radikale oder Protonen angebunden werden kann, welche bevorzugt nach einer Plasma- oder Koronabehandlung der Materialoberfläche entstehen und/oder eine perfluorierte Verbindung mit mindestens einer funktionellen Gruppe ist, welche für die Cycloaddition geeignet ist.

8. Verfahren gemäß dem vorherigen Anspruch
**dadurch gekennzeichnet, dass**
die perfluorierten Verbindungen Perfluorcarbone (PFC) sind, bevorzugt ausgewählt aus der Gruppe enthaltend C₁-C₁₀₀, bevorzugt C₁-C₅₀, insbesondere bevorzugt C₁-C₃₀, ganz bevorzugt C₁-C₂₀, höchst bevorzugt C₁-C₁₀ Alkane, Alkene oder Alkine, die linear, verzweigt, cyclisch, polycyclisch oder heterocyclisch sein können.

9. Verfahren gemäß einem der vorherigen Ansprüche
**dadurch gekennzeichnet, dass**
die funktionalisierten perfluorierten Verbindungen ausgewählt sind aus einer Gruppe enthaltend
- F(CF₂)ₙX, wobei n= 1-50, bevorzugt n = 1-10, und X = Br, I, Cl, H (H zweifle ich an, weil dann keine Abgangsgruppe da ist, H ist keine) oder X= Gruppen, die Si, N, O, S, P enthalten)
- F(CF₂)ₙ-(CH₂)ₘX, wobei n = 1-50, bevorzugt n = 1-10, m = 1-26, bevorzugt m = 1 - 6 und X = Si, N, O, S, P, Br, I, H; siehe oben
- F(CF₂)n-O_{b}-CH=CH₂ mit n = 1-50, bevorzugt n = 1-10, und b = 0 oder 1, bevorzugt b = 0.

10. Verfahren gemäß Anspruch 1
**dadurch gekennzeichnet, dass**
die perfluorierten Verbindungen Perfluorsiliziumverbindungen sind, bevorzugt ausgewählt aus der Gruppe enthaltend der Gruppe enthaltend Si₁-Si₁₀₀, bevorzugt Si₁-Si₅₀, insbesondere bevorzugt Si₁-Si₃₀, ganz bevorzugt Si₁-Si₂₀, höchst bevorzugt Si₁-S₁₀.

11. Verfahren gemäß einem der vorherigen Ansprüche 6 oder 7
**dadurch gekennzeichnet, dass**
die funktionalisierten Perfluorsiliziumverbindungen ausgewählt sind aus einer Gruppe enthaltend
- Siloxane der allgemeinen Formel -[-SiR₂-O-]ₙ- mit n≥1 und R = perfluorierte Carbone oder F,
- Siloxanole der allgemeinen Formel HO-A-[-SiR₃R₄-O-]-SiR₃R₄R₅ mit R_{3,4,5} = F oder -F(CF₂)ₙ, mit n = 1 - 10 und A = Alkylkette
- perfluorierte Silicate, Kieselsäuren, Natriumsilicate, Polysilazane, Silicide, Siliciumtetrahalogenide, Silikone, Silikonöle, Zeolithe, Zirkonsilikate.
- Silane der allgemeinen Formel X-CH₂-Si(O_{R})₃, wobei X eine für die nukleophile Substitution geeignete Abgangsgruppe mit X = Cl, Br, I, H, OH oder eine Gruppe umfassend Amino-, Vinyl-, Carbamato-, Glycidoxy-, Methylalkoxy-, Phenyl- oder Acetoxygruppen und R = F oder -F(CF₂)ₙ mit n = 1 - 10 ist,
oder
- Silane der allgemeinen Formel X-Si (CF₃)₃ oder X-Si(R)₃, wobei X eine für die nukleophile Substitution geeignete Abgangsgruppe mit X = Cl, Br, I, H, OH oder funktionellen Gruppen wie Amino-, Vinyl-, Carbamato-, Glycidoxy-, Methylalkoxy-, Phenyl- oder Acetoxygruppen und und R = F oder R = F(CF₂)ₙ mit n = 1 - 10 ist.

12. Verfahren gemäß einem der vorherigen Ansprüche
**dadurch gekennzeichnet, dass**
- die Materialoberfläche eine Keramikoberfläche ist, welche von einer Keramik gebildet wird, ausgewählt aus einer Gruppe enthaltend Aluminiumoxid, Zirkoniumoxid, Aluminiumtitanat, Siliziumkarbid, Siliziumnitrid oder Aluminiumnitrid oder andere nichtmetallische oder metallische Stoffe oder Dispersionskeramiken und Piezokeramiken, Porzellan, Steinzeug, Stealit, Glaskeramik, Cordlerit, Titanoxid, Yttriumoxid, Berylliumoxid, Magnesiumoxid, Uranoxid, Titanate. Bleititanalzirkonat, Ferrite, Kohlenstoff, Nitride, Carbide, Boride, Silicide (Silicium, Bor, Titan, Molybdän), wobei Hochleistungskeramiken insbesondere Aluminiumoxid, Zirkoniumoxid, Titanoxid, Aluminiumtitanat, Siliziumkarbid, Siliziumnitrid oder Aluminiumnitrid besonders bevorzugt sind,
- die Materialoberfläche eine Metalloberfläche ist, welche von einem Metall gebildet wird, ausgewählt aus einer Gruppe enthaltend Titan, Aluminium, Cadmium, Chrom, Eisen, Gold, Iridium, Cobalt, Kupfer, Nickel, Palladium, Platin, Silber, Zink und Zinn sowie Legierungen welche diese enthalten wie beispielsweise Titanlegierungen oder Edelstahl, ganz besonders bevorzugt wird die Metalloberfläche von Titan oder einer Titanlegierungen gebildet, und/oder
- die Materialoberfläche eine Kunststoffoberfläche ist, welche von einem Kunststoff gebildet wird, ausgewählt aus einer Gruppe enthaltend enthaltend Polyacetale, Polyacrylate, Polyamide, Polyaryle, Cellulosen, Polyester, Polyoleofine, Polystyrole, Polyvinyacatate, Polyvinylchloride, Polyetherketone, Polyaryletherketone, Polylactide, Aminoharze, Epoxidharze, Polyetheralkohole, Butadienelastomere, Fluorocarbonelastomere, Esterelastomere, Isoprenelastomere, Olefinelastomere, Siloconelastomere, Pentene-Elastomere, Sulfidelastomere, Urethanelastomere und Vinylchloridelastomere.

13. Inertisierte Materialoberfläche herstellbar durch ein Verfahren gemäß einem der vorherigen Ansprüche
**dadurch gekennzeichnet, dass**
die perfluorierte Verbindungen kovalent, durch lonenbeziehung oder durch Metallbindung an der Materialoberfläche gebunden vorliegen.

14. Inertisierte Materialoberfläche gemäß dem vorherigen Anspruch
**dadurch gekennzeichnet, dass**
die kovalente Bindung durch ein Linker- oder Spacermoleküle erfolgt, wobei das Linker- oder Spacermolekül bevorzugt auf Hydroxyl- oder Aminogruppen, Carboxylgruppen, Ester, Ether, Thioether, Thioester, Carbonsäureamide, Verbindungen mit Mehrfachbindungen, Carbamate, Disulfidbrücken und Hydrazide, Halogenalkane, Sulhydryl-, Aldehyd- Keto-, Carboxyl-, Ester- und Säureamidgruppen, Thiolen, Aminen, Iminen, Hydrazinen, oder Disulfidgruppen, Glycerol, Succinylglycerol, Orthoester, Phosphorsäurediester und Vinylether basiert, wobei Ester- und Ethergruppen und Disulfidbrücken ganz besonders bevorzugt sind.

15. Verwendung einer inertisierten Materialoberfläche gemäß einem der Ansprüche 13 oder 14
- in einem Medizinprodukt, bevorzugt in einem Implantat, besonders bevorzugt in einem Implantat, in dem eine Zellanlagerung ganz oder partiell unerwünscht ist, in einem Herzimplantat, einem Stent, einer Prothese, einem Gelenk, in Zahnimplantaten oder in einer Medizintechnikapparatur durch welche Substanzen, insbesondere Blut oder Blutbestandteile geleitet werden,
- in einem Produkt der Elektrotechnik, insbesondere der Mikroelektronik oder
- in Fenstergläsern, Einrichtungsgegenständen, sanitären Anlagen oder Gebäuden.
